# EUROPEAN PATENT APPLICATION

(11) **EP 2 045 266 A1**
(43) Date of publication of application: **08.04.2009**
(21) Application number: 07301421.9
(22) Date of filing: 02.10.2007
(51) Int. Cl.: C07K 14/70

(54) **Methods for treating and diagnosing a cancer secreting cath-D or Alzheimer's disease.**

(71) Applicant: INSERM (Institut National de la Santé et de la Recherche Médicale), 75654 Paris Cedex 13 (FR)
(72) Inventor: Liaudet, Emmanuelle, 34830 Clapiers (FR); Prebois, Christine, 34660 Cournonterral (FR); Derocq, Danielle, 34570 Murviel les Montpellier (FR); Beaujoin, Mélanie, 34090 Montpellier (FR)
(74) Representative: Niemann, Frédéric

(57) **Abstract**

The present invention relates to an inhibitor of the interaction between procath-D and LRP1 β chain and/or of LRP1 expression for the treatment and/or the prevention of a cancer secreting cath-D or of Alzheimer's disease.
The present invention also relates to the use of a fragment of LRP1 as a marker of a cancer secreting cath-D or of Alzheimer's disease.

## Description

### FIELD OF INVENTION

The present invention relates to methods for the treatment and/or the prevention of cancer and Alzheimer's disease.

The present invention also relates to an early marker of cancer in order to ensure its early diagnosis.

### BACKGROUND OF THE INVENTION

Cancer remains a major public health challenge despite progress in detection and therapy. Amongst the various types of cancer, breast cancer is worldwide the most common form of cancer in females and according to the United Nations World Health Organization, it is the leading cause of cancer deaths among women in 2005. Thus, breast cancer has led to the death of more than 500 000 people worldwide. Furthermore the number of cases has significantly increased since the 1970s, a phenomenon partly blamed on modern lifestyles in the Western world.

Breast cancer therefore remains incontestably a major human health problem necessitating urgently an effective treatment for this frequent and mortal pathology. Actually, the mainstay of breast cancer treatment is surgery when the tumor is localized, with possible adjuvant hormonal therapy (with, e.g., tamoxifen or an aromatase inhibitor), chemotherapy, and/or radiotherapy. For example, chemotherapy can be given both before and after surgery. Neo-adjuvant chemotherapy is used to shrink the size of a tumor prior to surgery. Adjuvant chemotherapy is given after surgery to reduce the risk of recurrence. Patients with estrogen receptor positive tumors will typically receive a hormonal treatment after chemotherapy is completed. But targeted therapies exist too. In patients whose cancer expresses an over-abundance of the HER2 protein the drug trastuzumab (Herceptin ®) is used to block the HER2 protein in breast cancer cells, thereby slowing their growth.

However despite all these therapeutic molecules, there remains an important medical need since breast cancer still leads to the death of half a million of people worldwide every year. Moreover breast cancer can originate from different genetic base and deregulation of biological pathways. It seems very important to determine new targets involved in apparition and development of breast cancer, notably at early steps. Metastasis can further emerge from this development of breast cancer. Thus, identification of new therapeutic targets is necessary in order to resolve problems of breast cancer and associated disorders.

It results from numerous scientific data that amongst the different therapeutic targets usable in the framework of breast cancer, lysosomal aspartic protease cathepsin-D (cath D) seems to can play a great role.

Cath-D is synthesized as the 52 kDa, catalytically inactive, precursor called pro-Cath-D. It is present in endosomes as an active 48 kDa single-chain intermediate that is subsequently converted in the lysosomes into the fully active mature protease, composed of a 34 kDa heavy and a 14 kDa light chains (Richo and Conner, 1991). In breast cancer cell lines, the 52 kDa pro-cath-D is hyper-secreted into the extracellular environment and can be taken up by both cancer cells and fibroblasts via mannose-6-phosphate (M6P) receptors and other as yet unidentified receptor(s) (Laurent-Matha et al., 1998; Heylen et al., 2002; Laurent-Matha et al., 2005).

The inventors have previously demonstrated that cath-D is over-expressed and secreted at high levels by human epithelial breast cancer cells (Liaudet-Coopman et al, 2006). Cath-D overexpression in breast cancer is highly correlated with poor prognosis (Ferrandina et al., 1997; Foekens et al., 1999; Bossard et al., 2003; Rodriguez et al., 2005). It stimulates cancer cell proliferation, fibroblast invasive growth and tumor angiogenesis and increases metastatic potential (Vignon et al., 1986; Garcia et al., 1990; Vetvicka et al., 1994; Liaudet et al., 1995; Glondu et al., 2001; Berchem et al., 2002; Laurent-Matha et al., 2005; Glondu et al., 2002). A mutant version of cath-D (D231 N) that has lost catalytic activity remains mitogenic for tumor cells and fibroblasts and still stimulates tumor angiogenesis (Glondu et al., 2001; Berchem et al., 2002; Laurent-Matha et al., 2005). These observations suggest that cath-D could act as an extracellular messenger that interacts either directly or indirectly, with an as yet unidentified cell surface receptor (Liaudet-Coopman et al, 2006). Cath-D is thus a marker of poor prognosis in cancer and its overexpression in cancer, notably breast cancer indicates a high risk of developing metastasis.

In the past and until today, important efforts have been devoted by many academic and private research laboratories in order to determine by which receptor is transduced the biological effect of Cath-D, and notably its mitogenic effect, involved in breast cancer for the major reason that identification of such receptor could permit to carry out an effective and expected therapeutic approach for ensuring a prevention and/or treatment of cancer overexpressing cath-D such breast cancer.

In parallel to this therapeutic aspect for cancer, an important need also relates to diagnostic of such cancer. Regardless of its origin, cancer morbidity increases significantly if it is not detected early in its progression. It results that a serum tumor marker which has acceptable sensitivity and specificity would be more suitable for screening than established methods; for example early diagnosis of cancer and notably breast cancer leading to a much better prognosis and a much better treatment. Thus, considerable efforts have focused on the elucidation of early cellular events surrounding transformation in breast tissue. Such efforts have led to the identification of several potential breast cancer markers. For example, alleles of the BRCA1 and BRCA2 genes have been linked to hereditary and early-onset breast cancer (EP 0 699 754, EP 0 705 902 and EP 0 785 216).

### SUMMARY OF THE INVENTION

The invention relates to an inhibitor of the interaction between pro-cath-D and the LRP1 β chain for the treatment and/or the prevention of a cancer secreting cath-D or of Alzheimer's disease, wherein said inhibitor is selected from the group consisting of a peptide having at least 80% sequence identity with SEQ ID NO: 5, an anti-LRP1 β chain antibody, an antibody fragment which binds to the LRP1 β chain, and an aptamer which binds to the LRP1 β chain.

The invention also relates to an inhibitor of the expression of LRP1 for the treatment and/or the prevention of a cancer secreting cath-D or of Alzheimer's disease.

A further embodiment of the invention relates to an in vitro method for diagnosing a cancer secreting cath-D or Alzheimer's disease in a patient comprising detecting the presence of a fragment of LRP1 in a biological sample obtained from said patient.

A further embodiment of the invention relates to a method for the *in vitro* screening of compounds that inhibit the interaction between pro-cath-D and

LRP1 β chain or a fragment thereof.

### DETAILED DESCRIPTION OF THE INVENTION

### Definitions

The term "pro-cath-D" is used herein to define the polypeptide sequence of the 52 kDa, catalytically inactive, precursor of cath-D. The naturally occurring protein has an aminoacid sequence shown in Genbank, Accession number NM_001909.

As intended herein, the term "LRP1" has its general meaning in the art (Strickland and Ranganathan, 2003; Lillis et al., 2005) and refers to LDL receptor-related protein 1. Thus, LRP1 is composed of a 515 kDa extra-cellular α chain and an 85 kDa β chain generated by proteolytic cleavage from a 600 kDa precursor polypeptide in a trans-Golgi compartment. Actually, LRP1 α chain and LRP1 β chain are issued from a sole transcript. By way of example, the human full length of unprocessed precursor LRP1 corresponds to SwissProt accession number Q07954.

The term "LRP1 β chain" is used herein to define the polypeptide sequence of the β chain of LRP1 consisting of the amino sequence as shown in SEQ ID NO: 1. The LRP1 β chain contains an extra-cellular domain, a trans-membrane region and a cytoplasmic tail.

The term "LRP1 β chain ectodomain" is used herein to define the polypeptide sequence of about 68kDa amino-terminal fragment of the extracellular domain of the LRP1 β chain. The LRP1 β chain ecto-domain corresponds to the liberated ectodomain of LRP1 β chain following to its cleavage by mature cath-D. By way of example the LRP1 β chain ecto-domain is represented by SEQ ID NO: 2.
Thus, the above-mentioned cleavage of LRP1 β chain ecto-domain by cath-D also leads to a 25 kDa membrane-associated carboxy-terminal fragment of the intracellular domain of the LRP1 β chain.
It must be noted that the full length extra-cellular domain of LRP1 β chain consists in 476 residues and that full length of LRP1 consists in 4544 amino acids.

As used herein, the expression "F0" relates to the polypeptide sequence of 173 amino acids corresponding to residues 307 to 479 of the extra-cellular domain of LRP1 β chain, which forms the pro-cath-D binding site on LRP1 β chain with a strong interaction. Thus, the residues 307 to 479 of the extra-cellular domain of LRP1 β chain correspond to the residues 4250 to 4422 of the full length of LRP1. By way of example, F0 is represented by SEQ ID NO: 3.
As used herein, the expression "F4" relates to the polypeptide sequence of 46 amino acids issued from "F0" and corresponding to the minimal pro-cath-D binding region on LRP1 β chain which consists in residues 349 to 394 of the extra-cellular domain of LRP1 β chain. Thus, the residues 349 to 394 of the extra-cellular domain of LRP1 β chain correspond to the residues 4292 to 4337 of the full length of LRP1. By way of example F4 is represented by SEQ ID NO: 4.
As used herein, the expression "49-mer" relates to the polypeptide sequence of 49 amino acids corresponding to the 349-397 fragment of the extra-cellular LRP1 β chain which competes efficiently for the binding to pro-cath-D with LRP1 β chain. Thus, this 49-mer is considered as an inhibitor of the interaction between pro-cath-D and LRP1 β chain. The residues 349 to 397 of the extra-cellular domain of LRP1 β chain correspond to the residues 4292 to 4340 of the full length of LRP1.

The term "inhibiting the interaction" or "inhibitor of the interaction" is used herein to mean preventing or reducing the direct or indirect association of one or more molecules, peptides, proteins, enzymes or receptors; or preventing or reducing the normal activity of one or more molecules, peptides, proteins, enzymes, or receptors.

The term "inhibitor of the interaction between pro-cath-D and LRP1 β chain" is a molecule which can prevent the interaction between pro-cath-D and LRP1 β chain by competition or by fixing to one of the molecule.

The term "expression" when used in the context of expression of a gene or nucleic acid refers to the conversion of the information, contained in a gene, into a gene product. A gene product can be the direct transcriptional product of a gene (e.g., mRNA, tRNA, rRNA, antisense RNA, ribozyme, structural RNA or any other type of RNA) or a protein produced by translation of a mRNA. Gene products also include messenger RNAs which are modified, by processes such as capping, polyadenylation, methylation, and editing, and proteins (e.g., LRP1) modified by, for example, methylation, acetylation, phosphorylation, ubiquitination, SUMOylation, ADP-ribosylation, myristilation, and glycosylation.
An "inhibitor of expression" refers to a natural or synthetic compound that has a biological effect to inhibit or significantly reduce the expression of a gene. Consequently an "inhibitor of LRP1 expression" refers to a natural or synthetic compound that has a biological effect to inhibit or significantly reduce the expression of the gene encoding for the LRP1 protein.
As used herein, the term "an inhibitor of LRP1 expression and/or interaction between pro-cath-D and LRP1 β chain" denotes a natural or synthetic compound which acts as an inhibitor of LRP1 expression and/or as an inhibitor of interaction between pro-cath-D and LRP1 β chain.
A "coding sequence" or a sequence "encoding" an expression product, such as an RNA, polypeptide, protein, or enzyme, is a nucleotide sequence that, when expressed, results in the production of that RNA, polypeptide, protein, or enzyme, i.e., the nucleotide sequence encodes an amino acid sequence for that polypeptide, protein or enzyme. A coding sequence for a protein may include a start codon (usually ATG) and a stop codon.
As used herein, references to specific proteins (e.g., LRP1) can include a polypeptide having a native amino acid sequence, as well as variants and modified forms regardless of their origin or mode of preparation. A protein that has a native amino acid sequence is a protein having the same amino acid sequence as obtained from nature (e.g., LRP1). Such native sequence proteins can be isolated from nature or can be prepared using standard recombinant and/or synthetic methods. Native sequence proteins specifically encompass naturally occurring truncated or soluble forms, naturally occurring variant forms (e.g., alternatively spliced forms), naturally occurring allelic variants and forms including postranslational modifications. A native sequence protein includes proteins following post-translational modifications such as glycosylation, or phosphorylation, or other modifications of some amino acid residues.
Variants refer to proteins that are functional equivalents to a native sequence protein that have similar amino acid sequences and retain, to some extent, one or more activities of the native protein. Variants also include fragments that retain activity. Variants also include proteins that are substantially identical (e.g., that have 80, 85, 90, 95, 97, 98, 99%, sequence identity) to a native sequence. Such variants include proteins having amino acid alterations such as deletions, insertions and/or substitutions. A "deletion" refers to the absence of one or more amino acid residues in the related protein. The term "insertion" refers to the addition of one or more amino acids in the related protein. A "substitution" refers to the replacement of one or more amino acid residues by another amino acid residue in the polypeptide. Typically, such alterations are conservative in nature such that the activity of the variant protein is substantially similar to a native sequence protein (see, e.g., Creighton (1984) Proteins, W.H. Freeman and Company). In the case of substitutions, the amino acid replacing another amino acid usually has similar structural and/or chemical properties. Insertions and deletions are typically in the range of 1 to 5 amino acids, although depending upon the location of the insertion, more amino acids can be inserted or removed. The variations can be made using methods known in the art such as site-directed mutagenesis (Carter, et al. 1985; Nucl. Acids Res. 13:4331; Zoller et al. 1982), cassette mutagenesis (Wells et al. 1985), and PCR mutagenesis (Sambrook et al., Molecular Cloning: A Laboratory Manual, 3rd edition, Cold Spring Harbor Press, N.Y., (2001)).
Two amino acid sequences are "substantially homologous" or "substantially similar" when greater than 80 %, preferably greater than 85 %, still preferably greater than 90 % of the amino acids are identical, or greater than about 90 %, preferably grater than 95 %, are similar (functionally identical) over the whole length sequences. Preferably, the similar or homologous sequences are identified by alignment using, for example, the GCG (Genetics Computer Group, Program Manual for the GCG Package, Version 7, Madison, Wisconsin) pileup program, or any of sequence comparison algorithms such as BLAST, FASTA, etc.
As intended herein, the expression "cancer" relates to a disease characterized by an uncontrolled cell growth and invasion of adjacent tissues. The abnormal cells often are referred to as neoplastic cells which are transformed cells that can form a solid tumor.
The term "tumor" refers to an abnormal mass or population of cells (i.e. two or more cells) that result from excessive or abnormal cell division, whether malignant or benign, and pre-cancerous and cancerous cells. Malignant tumors are distinguished from benign growths or tumors in that, in addition to uncontrolled cellular proliferation, they can invade surrounding tissues and can metastasize.
As used herein, the term "metastasis" refers to a process in which cancer cells spread from one organ or tissue to another non-adjacent organ or tissue.

The term "associated hyperproliferative disorders" means tumor microenvironment, fibroblast invasive outgrowth and metastasis development. Indeed, the tumor microenvironment was shown to play a critical role in tumor initiation and progression, and may be an important factor in developing therapeutic approaches. The tumor microenvironment, or stroma, influences the growth of the tumor and its ability to progress and metastasize.

As intended herein, the expression "cancer secreting cath-D" is meant cancer and associated hyperproliferative disorders in which cath-D or pro-cath-D are secreted by cancer cells. Different approaches, such as immuno-histochemistry, *in situ* hybridization, cytosolic immunoassay and Northern and Western blot analyses have indicated that in most breast cancer tumors, cath-D is over-expressed from 2- to 50-fold compared to its concentration in other cell types such as fibroblasts or normal mammary glands [from F. Capony, C. Rougeot, P. Montcourrier, V. Cavaillès, G. Salazar, H. Rochefort, Increased secretion, altered processing, and glycosylation of pro-cathepsin D in human mammary cancer cells, Cancer Res. 49 (1989) 3904-3909]. Examples of cancers secreting cath-D include, but are not limited to, breast cancer, stomach cancer (cf. Manuel Del Casar J, Vizoso FJ, Abdel-Laa O, Sanz L, Martin A, Daniela Corte M, Bongera M, Garcia Muniz JL, Fueyo A. Prognostic value of cytosolyc cathepsin D content in resectable gastric cancer. J Surg Oncol. 2004 86:16-21), liver cancer (cf. Ito H, Miyazaki M, Nishimura F, Nakajima N. Secretion of extracellular matrix (fibronectin), growth factor (transforming growth factor beta) and protease (cathepsin D) by hepatoma cells. Oncology. 2000 58:261-70), glioblastoma (cf. Fukuda ME, Iwadate Y, Machida T, Hiwasa T, Nimura Y, Nagai Y, Takiguchi M, Tanzawa H, Yamaura A, Seki N. Cathepsin D is a potential serum marker for poor prognosis in glioma patients. Cancer Res. 2005 65:5190-4. and Sivaparvathi M, Sawaya R, Chintala SK, Go Y, Gokaslan ZL, Rao JS. Expression of cathepsin D during the progression of human gliomas. Neurosci Lett. 1996 208:171-4), Melanoma (cf. Pardo M, Garcia A, Antrobus R, Blanco MJ, Dwek RA, Zitzmann N. Biomarker discovery from uveal melanoma secretomes: identification of gp100 and cathepsin D in patient serum. J Proteome Res. 2007 6:2802-11 and Podhajcer OL, Bover L, Bravo Al, Ledda MF, Kairiyama C, Calb I, Guerra L, Capony F, Mordoh J. Expression of cathepsin D in primary and metastatic human melanoma and dysplastic nevi. J Invest Dermatol. 1995 104:340-4), Squamous cell carcinoma (SCC) (cf. Vigneswaran N, Zhao W, Dassanayake A, Muller S, Miller DM, Zacharias W. Variable expression of cathepsin B and D correlates with highly invasive and metastatic phenotype of oral cancer. Hum Pathol. 2000 31:931-7 and Kawasaki G, Kato Y, Mizuno A. Cathepsin expression in oral squamous cell carcinoma: relationship with clinicopathologic factors. Oral Surg Oral Med Oral Pathol Oral Radiol Endod. 2002 93:446-54), Ovarian cancer (cf. Losch A, Schindl M, Kohlberger P, Lahodny J, Breitenecker G, Horvat R, Birner P. Cathepsin D in ovarian cancer: prognostic value and correlation with p53 expression and microvessel density. Gynecol Oncol. 2004 92:545-52), Pancreatic cancer (cf. Whiteman HJ, Weeks ME, Dowen SE, Barry S, Timms JF, Lemoine NR, Crnogorac-Jurcevic T. The Role of S100P in the Invasion of Pancreatic Cancer Cells Is Mediated through Cytoskeletal Changes and Regulation of Cathepsin D.Cancer Res. 2007 67:8633-42, and Shen J, Person MD, Zhu J, Abbruzzese JL, Li D. Protein expression profiles in pancreatic adenocarcinoma compared with normal pancreatic tissue and tissue affected by pancreatitis as detected by two-dimensional gel electrophoresis and mass spectrometry. Cancer Res. 2004 64:9018-26).

As used herein, the term "breast cancer" refers to a condition characterized by anomalous rapid proliferation of abnormal cells in one or both breasts of a subject. In breast cancer, neoplastic cells may be identified in one or both breasts only and not in another tissue or organ, in one or both breasts and one or more adjacent tissues or organs (e.g. lymph node), or in a breast and one or more non-adjacent tissues or organs to which the breast cancer cells have metastasized. Cancer cells in the breast spread to tissues and organs of a subject, and conversely, cancer cells from other organs or tissue can invade or metastasize to a breast. Breast cancer cells often invade lymph node cells and/or metastasize to the liver, brain and/or bone and spread cancer in these tissues and organs. Breast cancers can spread to other organs and tissues and cause lung cancer, prostate cancer, colon cancer, ovarian cancer, cervical cancer, gastrointestinal cancer, pancreatic cancer, glioblastoma, hepatoma, colorectal cancer, uterine cervical cancer, endometrial carcinoma, salivary gland carcinoma, kidney cancer, vulval cancer, thyroid cancer, hepatic carcinoma, skin cancer, melanoma, ovarian cancer, neuroblastoma, myeloma, Ewing sarcoma and peripheral neuroepithelioma, and other carcinomas, lymphomas, blastomas, sarcomas, and leukemias.

In its broadest meaning, the term "treating" or "treatment" refers to reversing, alleviating, inhibiting the progress of, or preventing the disorder or condition to which such term applies, or one or more symptoms of such disorder or condition.

The term "diagnosis" as used herein means the process of identifying a medical condition or disease by its signs and symptoms and from the results of various diagnostic procedures. For the purpose of the present invention, "diagnosis" is used to mean determining the incidence of a cancer secreting cath-D, for example breast cancer, by examining whether the diagnostic marker of cancer secreting cath-D is present in a sample obtained from a patient.

As used herein, the term "marker" or "biochemical marker" as used herein refers to a molecule used as a target for analyzing patient test samples.

As used herein, the term "biological sample" as used herein refers to any biological sample obtained for the purpose of evaluation in vitro. In the methods of the present invention, the sample or patient sample may comprise any body fluid. Examples of test samples include blood, serum, plasma, nipple aspirate fluid, urine, saliva, synovial fluid and cephalorachidian liquid (CRL). Preferably the sample is a whole blood sample, a serum sample or a plasma sample.

As used herein, the term "patient" denotes a mammal, such as a rodent, a feline, a canine, and a primate. Preferably, a patient according to the invention is a human.

### Therapeutic methods and uses

The inventors have demonstrated that LRP1 plays a major role in the development of cancer and associated hyperproliferative disorders, notably cancer secreting cath-D such as breast cancer. Hence, a new pathway for the treatment and/or the prevention of cancer secreting cath-D is provided which involves inhibiting the interaction between pro-cath-D and the β chain of LRP1 and/or inhibiting LRP1 expression.
The present application also shows the usefulness of LRP1 fragments and more particularly LRP1 β chain ecto-domain in diagnosis of a cancer secreting cath-D cancer such as breast cancer.
The inventors have indeed demonstrated that pro-cath-D is a ligand for the extracellular domain of the β chain of LDL receptor-related protein-1, LRP1. Until this demonstration, no ligand has been described that binds directly to the extra-cellular domain of the β chain. The inventors have observed the physical interaction between pro-cath-D and the extra-cellular domain of LRP1 β chain.
The inventors have also underlined that in the context of the micro-environment present in cancer secreting cath-D such as breast cancer, cath-D participates in the paracrine signalling between epithelial cancer cells and associated fibroblasts, a process that depends on the presence of LRP1 on the surface of the fibroblasts.
Thus the present invention arises from the identification by the inventors of a key component of the tumor environment, namely LRP1, which is critical for the development, progression and metastasis of cancer and thus proposes a therapeutic strategy to target the tumor microenvironment by the inhibition of the function of the stromal cells that are required for progression. The inhibitors according to the invention enable to block the function of LRP1 as demonstrated by the inventors, which are responsible for tumor cell survival and proliferation.

The inventors have also shown cath-D causes shedding of the extracellular domain of the LRP1 β chain so called LRP1 β chain ecto-domain, resulting in the generation of an amino-terminal fragment of about 68 kDa.
This cleaved extracellular domain of the LRP1 β chain, the LRP1 α chain and fragments thereof may be detected in the circulation of patients affected by a cancer secreting cath-D.
Thus, such fragment of LRP1 β chain can be used for the detection of cancer secreting cath-D and in a preferred embodiment for early detection of breast cancer.
The present invention provides methods and compositions (such as pharmaceutical compositions) for treating a cancer secreting cath-D, breast cancer in particular.

Furthermore the inventors identified an analogy between the mechanism of action cath-D in cancers secreting cath-D and the mechanism of action cath-D in Alzheimer's disease. In Alzheimer's disease, the beta and gamma secretases are highly specific proteases that cleave the amyloid peptide (Abeta) from its protein precursor, amyloid precursor protein (APP). Overproduction of Abeta and its subsequent aggregation into amyloid plaques is thought to be the initial step in the pathological process that leads to Alzheimer's disease.
The beta secretase clips APP near the transmembrane domain, releasing a large soluble fragment (beta-ectodomain); the smaller fragment is subsequently cleaved by gamma secretases to generate Abeta in a process called RIP.
In the brain, the identified beta secretases are the aspartic proteases BACE1, BACE2 (a BACE homolog), cathepsin D and cathepsin E.
LRP1 was shown to interact with APP. LRP1 facilitates APP processing via the amyloidogenic pathway and the production of Abeta. Evidence that LRP1 is involved in APP processing is based on the findings that APP can interact with LRP1. Direct interaction between the extracellular KPI domain of APP and ligand binding domains of LRP1, as well as indirect interaction between APP and LRP1 cytoplasmic tails bridged by the adaptor protein FE65, have been previously shown to occur in vitro. It was proposed that upon interaction between LRP and APP at the cell surface, the fast endocytosis rate of LRP1 may accelerate APP trafficking within endosomes and therefore increase Abeta production.
Whether the proteolytical processing of LRP1 itself plays a role in this regulatory process has not been examinated so far. However it was recently found that the protease BACE1, which generates the Abeta N-terminus through the cleavage of APP, is also able to exert the extracellular cleavage of LRP1, which further links the processing of the two proteins.
It is therefore credible that cath-D, that also exerts extracellular cleavage of LRP1, influences the processing of APP and the production of Abeta.

Thus, an object of the invention is the use of an inhibitor of LRP1 expression and/or interaction between pro-cath-D and LRP1 β chain for the manufacture of a medicament for preventing and/or treating cancer cancer secreting cath-D or Alzheimer's disease.

According to a first aspect, the invention relates to an inhibitor of the interaction between pro-cath-D and LRP1 β chain for the treatment and/or the prevention of a cancer secreting cath-D or of Alzheimer's disease.
In one embodiment, the inhibitor of the interaction between pro-cath-D and LRP1 β is a peptide having at least 80%, preferably at least 90%, even more preferably at least 95% sequence identity with the sequence of SEQ ID NO: 5. Typically the length of said peptide is less than 60, preferably less than 55, amino acid residues. SEQ ID NO: 5 represents the sequence of the polypeptides called "49-mer" defined by the sequence of amino acid between 349 and 397 of the LRP1 β chain.

In another embodiment the inhibitor of the interaction between pro-cath-D and LRP1 β chain is an antibody or a fragment thereof which binds to the β of chain LRP1.
In a preferred embodiment, the antibody or the fragment thereof is directed against the polypeptide F0 or F4.
In a more preferred embodiment, the antibody is directed against the sequence of amino acid between 349 and 397 of LRP1 β chain consisting in the SEQ ID NO: 5.
Examples of antibodies include, but are not limitated to, the monoclonal anti-α2 macroglobulin receptor (# 3501, American Diagnostica Inc), the polyclonal LRP1 (N-20) (sc-16166, Santa Cruz Biotechnology, Inc.) and the monoclonal LRP1 (8B8) (sc-57352, Santa Cruz Biotechnology, Inc.).
The monoclonal anti-α2 macroglobulin receptor (# 3501, American Diagnostica Inc) recognizes amino acids 4291-4344 of LRP1. In another preferred embodiment of the present invention the inhibitor of the interaction between pro-cath-D and LRP1 β chain is an antibody or antibody fragment that is directed against pro-cath-D and preferably against epitopes issued from LPR1 β chain binding site on pro-cath-D.
Epitopes issued form pro-cath-D binding region on LRP1 β chain can be characterized by the fact that each catalytic aspartic acid, namely Asp 33 on 14 kDa subunit and Asp 231 on 34 kDa subunit, have a central position in such epitopes.

Antibodies directed against the LRP1 β chain can be raised according to known methods by administering the appropriate antigen or epitope to a host animal selected, e.g., from pigs, cows, horses, rabbits, goats, sheep, and mice, among others. Various adjutants known in the art can be used to enhance antibody production. Although antibodies useful in practicing the invention can be polyclonal, monoclonal antibodies are preferred. Monoclonal antibodies against LRP1 β chain or ligands of LRP1 β chain can be prepared and isolated using any technique that provides for the production of antibody molecules by continuous cell lines in culture. Techniques for production and isolation include but are not limited to the hybridoma technique originally described by Kohler and Milstein (1975); the human B-cell hybridoma technique (Cote et al., 1983); and the EBV-hybridoma technique (Cole et al. 1985). Alternatively, techniques described for the production of single chain antibodies (see, e.g., U.S. Pat. No. 4,946,778) can be adapted to produce anti-LRP1 β chain, or anti-LRP1 β chain ligands single chain antibodies. Inhibitors of the interaction between pro-cath-D and LRP1 β chain useful in practicing the present invention also include anti-LRP1 β chain, or anti-LRP1 β chain ligands antibody fragments including but not limited to F(ab')₂ fragments, which can be generated by pepsin digestion of an intact antibody molecule, and Fab fragments, which can be generated by reducing the disulfide bridges of the F(ab')₂ fragments. Alternatively, Fab and/or scFv expression libraries can be constructed to allow rapid identification of fragments having the desired specificity to LRP1 β chain.
Humanized anti-LRP1 β chain or anti-LRP1 β chain ligands antibodies and antibody fragments therefrom can also be prepared according to known techniques. "Humanized antibodies" are forms of non-human (e.g., rodent) chimeric antibodies that contain minimal sequence derived from non-human immunoglobulin. For the most part, humanized antibodies are human immunoglobulins (recipient antibody) in which residues from a hypervariable region (CDRs) of the recipient are replaced by residues from a hypervariable region of a non-human species (donor antibody) such as mouse, rat, rabbit or nonhuman primate having the desired specificity, affinity and capacity. In some instances, framework region (FR) residues of the human immunoglobulin are replaced by corresponding non-human residues. Furthermore, humanized antibodies may comprise residues that are not found in the recipient antibody or in the donor antibody. These modifications are made to further refine antibody performance. In general, the humanized antibody will comprise substantially all of at least one, and typically two, variable domains, in which all or substantially all of the hypervariable loops correspond to those of a non-human immunoglobulin and all or substantially all of the FRs are those of a human immunoglobulin sequence. The humanized antibody optionally also will comprise at least a portion of an immunoglobulin constant region (Fc), typically that of a human immunoglobulin. Methods for making humanized antibodies are described, for example, by Winter (U.S. Pat. No. 5,225,539) and Boss (Celltech, U.S. Pat. No. 4,816,397).
In still another embodiment, use may be made of aptamers.
Aptamers are a class of molecule that represents an alternative to antibodies in term of molecular recognition. Aptamers are oligonucleotide or oligopeptide sequences with the capacity to recognize virtually any class of target molecules with high affinity and specificity. Such ligands may be isolated through Systematic Evolution of Ligands by EXponential enrichment (SELEX) of a random sequence library, as described in Tuerk C. and Gold L., 1990. The random sequence library is obtainable by combinatorial chemical synthesis of DNA. In this library, each member is a linear oligomer, eventually chemically modified, of a unique sequence. Possible modifications, uses and advantages of this class of molecules have been reviewed in Jayasena S.D., 1999. Peptide aptamers consists of a conformationally constrained antibody variable region displayed by a platform protein, such as E. coli Thioredoxin A that are selected from combinatorial libraries by two hybrid methods (Colas et al., 1996).
A further aspect of the invention related to nucleic acids encoding for polypeptides of the invention, notably F0, F4 and the peptide named 49-mer.
Typically, said nucleic acid construct is a DNA or RNA molecule, which may be included in any suitable vector, such as a plasmid, cosmid, episome, artificial chromosome, phage or a viral vector.
So a further aspect of the invention relates to a vector comprising a nucleic acid of the invention.
Such vectors may comprise regulatory elements, such as a promoter, enhancer, terminator and the like, to cause or direct expression of said polypeptide upon administration to a subject. The vectors may further comprise one or several origins of replication and/or selectable markers. The promoter region may be homologous or heterologous with respect to the coding sequence, and provide for ubiquitous, constitutive, regulated and/or tissue specific expression, in any appropriate host cell, including for in vivo use. Examples of promoters include bacterial promoters (T7, pTAC, Trp promoter, etc.), viral promoters (LTR, TK, CMV-IE, etc.), mammalian gene promoters (albumin, PGK, etc), and the like.
Examples of plasmids include replicating plasmids comprising an origin of replication, or integrative plasmids, such as for instance pUC, pcDNA, pBR, and the like. Examples of viral vector include adenoviral, retroviral, herpesvirus and AAV vectors. Such recombinant viruses may be produced by techniques known in the art, such as by transfecting packaging cells or by transient transfection with helper plasmids or viruses. Typical examples of virus packaging cells include PA317 cells, PsiCRIP cells, GPenv+ cells, 293 cells, etc. Detailed protocols for producing such replication-defective recombinant viruses may be found for instance in WO95/14785, WO96/22378, US5,882,877, US6,013,516, US4,861,719, US5,278,056 and WO94/19478.
A further aspect of the present invention resides in a cell which has been transfected, infected or transformed by a nucleic acid and/or a vector according to the invention.
Suitable host cells include, without limitation, prokaryotic cells (such as bacteria) and eukaryotic cells (such as yeast cells, mammalian cells, insect cells, plant cells, etc.). Specific examples include E.coli, Kluyveromyces or Saccharomyces yeasts, mammalian cell lines (e.g., Vero cells, CHO cells, 3T3 cells, COS cells, etc.) as well as primary or established mammalian cell cultures (e.g., produced from lymphoblasts, fibroblasts, embryonic cells, epithelial cells, nervous cells, adipocytes, etc.). More particularly, the invention contemplates vascular or endothelial cells thereof or derived thereof, such as human umbilical vein endothelial (HUVEC) or progenitor endothelial cells (PEC).
The present invention also relates to a method for producing a recombinant host cell expressing a polypeptide according to the invention, said method comprising (i) introducing *in vitro* or *ex vivo* into a competent host cell a recombinant nucleic acid or a vector as described above, (ii) culturing *in vitro* or *ex vivo* the recombinant host cells obtained and (iii), optionally, selecting the cells which express and/or secrete said polypeptide. Such recombinant host cells can be used for the production of polypeptides according to the present invention as previously described.

According to another aspect, the invention relates to an inhibitor of the expression of LRP1 for the treatment and/or the prevention of a cancer secreting cath-D or of Alzheimer's disease.
Thus, the invention provides the use of an inhibitor of LRP1 expression for the manufacture of a medicament intended for preventing and/or treating a cancer secreting cath-D or Alzheimer's disease.

Inhibitors of LRP1 expression according to the present invention may be based on anti-sense oligonucleotide constructs. Anti-sense oligonucleotides, including anti-sense RNA molecules and anti-sense DNA molecules, would act to directly block the translation of LRP1 mRNA by binding thereto and thus preventing protein translation or increasing mRNA degradation, thus decreasing the level of LRP1 (and finally LRP1 β chain), and thus activity, in a cell. For example, antisense oligonucleotides of at least about 15 bases and complementary to unique regions of the mRNA transcript sequence encoding LRP1 can be synthesized, e.g., by conventional phosphodiester techniques and administered by e.g., intravenous injection or infusion. Methods for using antisense techniques for specifically inhibiting gene expression of genes whose sequence is known are well known in the art (e.g. see U.S. Pat. Nos. 6,566,135; 6,566,131; 6,365,354; 6,410,323; 6,107,091; 6,046,321; and 5,981,732).
Small inhibitory RNAs (siRNAs) can also function as inhibitors of LRP1 expression for use in the present invention. LRP1 expression can be reduced by contacting a subject or cell with a small double stranded RNA (dsRNA), or a vector or construct causing the production of a small double stranded RNA, such that LRP1 expression is specifically inhibited (i.e. RNA interference or RNAi). Methods for selecting an appropriate dsRNA or dsRNA-encoding vector are well known in the art for genes whose sequence is known (e.g. see Tuschl, T. et al. (1999); Elbashir, S. M. et al. (2001); Hannon, GJ. (2002); McManus, MT. et al. (2002); Brummelkamp, TR. et al. (2002); U.S. Pat. Nos. 6,573,099 and 6,506,559; and International Patent Publication Nos. WO 01/36646, WO 99/32619, and WO 01/68836).
By way of example, a siRNA useful within the context of the present invention consists in duplexes of 21-nucleotide human LRP1 siRNA (target sequence AAGCAGTTTGCCTGCAGAGAT (SEQ ID NO: 6), residues 666-684) (Li et al., 2003).
Ribozymes can also function as inhibitors of LRP1 expression for use in the present invention. Ribozymes are enzymatic RNA molecules capable of catalyzing the specific cleavage of RNA. The mechanism of ribozyme action involves sequence specific hybridization of the ribozyme molecule to complementary target RNA, followed by endonucleolytic cleavage. Engineered hairpin or hammerhead motif ribozyme molecules that specifically and efficiently catalyze endonucleolytic cleavage of LRP1 mRNA sequences are thereby useful within the scope of the present invention. Specific ribozyme cleavage sites within any potential RNA target are initially identified by scanning the target molecule for ribozyme cleavage sites, which typically include the following sequences, GUA, GUU, and GUC. Once identified, short RNA sequences of between about 15 and 20 ribonucleotides corresponding to the region of the target gene containing the cleavage site can be evaluated for predicted structural features, such as secondary structure, that can render the oligonucleotide sequence unsuitable. The suitability of candidate targets can also be evaluated by testing their accessibility to hybridization with complementary oligonucleotides, using, e.g., ribonuclease protection assays.
Both antisense oligonucleotides and ribozymes useful as inhibitors of LRP1 expression can be prepared by known methods. These include techniques for chemical synthesis such as, e.g., by solid phase phosphoramadite chemical synthesis. Alternatively, anti-sense RNA molecules can be generated by in vitro or in vivo transcription of DNA sequences encoding the RNA molecule. Such DNA sequences can be incorporated into a wide variety of vectors that incorporate suitable RNA polymerase promoters such as the T7 or SP6 polymerase promoters. Various modifications to the oligonucleotides of the invention can be introduced as a means of increasing intracellular stability and half-life. Possible modifications include but are not limited to the addition of flanking sequences of ribonucleotides or deoxyribonucleotides to the 5' and/or 3' ends of the molecule, or the use of phosphorothioate or 2'-O-methyl rather than phosphodiesterase linkages within the oligonucleotide backbone.
Antisense oligonucleotides siRNAs and ribozymes of the invention may be delivered in vivo alone or in association with a vector. In its broadest sense, a "vector" is any vehicle capable of facilitating the transfer of the antisense oligonucleotide siRNA or ribozyme nucleic acid to the cells and preferably cells expressing LRP1. Preferably, the vector transports the nucleic acid to cells with reduced degradation relative to the extent of degradation that would result in the absence of the vector. In general, the vectors useful in the invention include, but are not limited to, plasmids, phagemids, viruses, other vehicles derived from viral or bacterial sources that have been manipulated by the insertion or incorporation of the the antisense oligonucleotide siRNA or ribozyme nucleic acid sequences. Viral vectors are a preferred type of vector and include, but are not limited to nucleic acid sequences from the following viruses: retrovirus, such as moloney murine leukemia virus, harvey murine sarcoma virus, murine mammary tumor virus, and rouse sarcoma virus; adenovirus, adeno-associated virus; SV40-type viruses; polyoma viruses; Epstein-Barr viruses; papilloma viruses; herpes virus; vaccinia virus; polio virus; and RNA virus such as a retrovirus. One can readily employ other vectors not named but known to the art.
Preferred viral vectors are based on non-cytopathic eukaryotic viruses in which non-essential genes have been replaced with the gene of interest. Non-cytopathic viruses include retroviruses (e.g., lentivirus), the life cycle of which involves reverse transcription of genomic viral RNA into DNA with subsequent proviral integration into host cellular DNA. Retroviruses have been approved for human gene therapy trials. Most useful are those retroviruses that are replication-deficient (i.e., capable of directing synthesis of the desired proteins, but incapable of manufacturing an infectious particle). Such genetically altered retroviral expression vectors have general utility for the high-efficiency transduction of genes in vivo. Standard protocols for producing replication-deficient retroviruses (including the steps of incorporation of exogenous genetic material into a plasmid, transfection of a packaging cell lined with plasmid, production of recombinant retroviruses by the packaging cell line, collection of viral particles from tissue culture media, and infection of the target cells with viral particles) are provided in Kriegler, 1990 and in Murry, 1991).
Preferred viruses for certain applications are the adeno-viruses and adeno-associated viruses, which are double-stranded DNA viruses that have already been approved for human use in gene therapy. The adeno-associated virus can be engineered to be replication deficient and is capable of infecting a wide range of cell types and species. It further has advantages such as, heat and lipid solvent stability; high transduction frequencies in cells of diverse lineages, including hemopoietic cells; and lack of superinfection inhibition thus allowing multiple series of transductions. Reportedly, the adeno-associated virus can integrate into human cellular DNA in a site-specific manner, thereby minimizing the possibility of insertional mutagenesis and variability of inserted gene expression characteristic of retroviral infection. In addition, wild-type adeno-associated virus infections have been followed in tissue culture for greater than 100 passages in the absence of selective pressure, implying that the adeno-associated virus genomic integration is a relatively stable event. The adeno-associated virus can also function in an extrachromosomal fashion.
Other vectors include plasmid vectors. Plasmid vectors have been extensively described in the art and are well known to those of skill in the art. See e.g. Sambrook et al., 1989. In the last few years, plasmid vectors have been used as DNA vaccines for delivering antigen-encoding genes to cells in vivo. They are particularly advantageous for this because they do not have the same safety concerns as with many of the viral vectors. These plasmids, however, having a promoter compatible with the host cell, can express a peptide from a gene operatively encoded within the plasmid. Some commonly used plasmids include pBR322, pUC18, pUCI9, pRC/CMV, SV40, and pBlueScript. Other plasmids are well known to those of ordinary skill in the art. Additionally, plasmids may be custom designed using restriction enzymes and ligation reactions to remove and add specific fragments of DNA. Plasmids may be delivered by a variety of parenteral, mucosal and topical routes. For example, the DNA plasmid can be injected by intramuscular, intradermal, subcutaneous, or other routes. It may also be administered by intranasal sprays or drops, rectal suppository and orally. It may also be administered into the epidermis or a mucosal surface using a gene-gun. The plasmids may be given in an aqueous solution, dried onto gold particles or in association with another DNA delivery system including but not limited to liposomes, dendrimers, cochleate and microencapsulation.

Another object of the invention relates to a method for preventing and/or treating a cancer secreting cath-D or Alzheimer's disease comprising administering to a patient in need thereof a therapeutically effective amount of an inhibitor of LRP1 expression and/or of an inhibitor of the interaction between pro-cath-D and LRP1 β chain as defined above.
The inhibitor of LRP1 expression and/or interaction between pro-cath-D and LRP1 β chain expression may be administered in the form of a pharmaceutical composition, as defined below.
By a "therapeutically effective amount" is meant a sufficient amount of the an inhibitor of LRP1 expression and/or a sufficient amount of the an inhibitor of interaction between pro-cath-D and LRP1 β chain to treat and/or to prevent a cancer secreting cath-D or Alzheimer's disease at a reasonable benefit/risk ratio applicable to any medical treatment.
Example of cancers secreting cath-are breast cancer, stomach cancer, liver cancer, glioblastoma, melanoma, squamous cell carcinoma, ovarian cancer and pancreatic cancer.
It will be understood that the total daily usage of the compounds and compositions of the present invention will be decided by the attending physician within the scope of sound medical judgment. The specific therapeutically effective dose level for any particular patient will depend upon a variety of factors including the disorder being treated and the severity of the disorder; activity of the specific compound employed; the specific composition employed, the age, body weight, general health, sex and diet of the patient; the time of administration, route of administration, and rate of excretion of the specific compound employed; the duration of the treatment; drugs used in combination or coincidental with the specific polypeptide employed; and like factors well known in the medical arts. For example, it is well within the skill of the art to start doses of the compound at levels lower than those required to achieve the desired therapeutic effect and to gradually increase the dosage until the desired effect is achieved. However, the daily dosage of the products may be varied over a wide range from 0.01 to 1,000 mg per adult per day. Preferably, the compositions contain 0.01, 0.05, 0.1, 0.5, 1.0, 2.5, 5.0, 10.0, 15.0, 25.0, 50.0, 100, 250 and 500 mg of the active ingredient for the symptomatic adjustment of the dosage to the patient to be treated. A medicament typically contains from about 0.01 mg to about 500 mg of the active ingredient, preferably from 1 mg to about 100 mg of the active ingredient. An effective amount of the drug is ordinarily supplied at a dosage level from 0.0002 mg/kg to about 20 mg/kg of body weight per day, especially from about 0.001 mg/kg to 7 mg/kg of body weight per day.

### Screening methods

Inhibitors of the invention can be identified by adapting screening methods described in the state of the art.
Thus, an aspect of the invention relates to a method for the *in vitro* screening of compounds that inhibit the interaction between pro-cath-D and LRP1 β chain or a fragment thereof wherein said method comprises the following steps:
a) determining the ability of a candidate compound to inhibit the interaction between pro-cath-D and LRP1 β chain or a fragment thereof, and
b) selecting the candidate compound that inhibits the interaction between pro-cath-D and LRP1 β chain or a fragment thereof.

At step a), any method suitable for the screening of protein-protein interactions is suitable.
Whatever the embodiment of step a) of the screening method, the complete pro-cath-D and the complete LRP1 β chain may be used as the binding partners.
Regarding to LRP1 β chain derived polypeptides, the inventors indeed have shown that the pro-cath-D and LRP1 β chain bind via specific regions of each complete protein. Actually, it has been found that LRP1 β chain interacts with pro-cath-D through its extra-cellular domain and more precisely through the polypeptide "F0" defined by the sequence of amino acid between 307 and 479 of LRP1 β chain as set forth in SEQ ID NO: 3.
Therefore in one embodiment step a) of the screening method of the invention consists of the following steps:
a1) bringing into contact the candidate compound to be tested with a mixture of:
   (1) pro-cath-D, and
   (2) the poplypeptide F0 or a substantially homologous or substantially similar amino acid sequence thereof
a2) determining the ability of said candidate compound to inhibit the binding between said pro-cath-D and F0.

The inventors have shown that the minimal binding region on LRP1 β chain of pro-cath-D consist in the polypeptide "F4" defined by the sequence of amino acid between 349 and 394 of LRP1 β chain as set forth in SEQ ID NO: 4.
Therefore in one embodiment step a) of the screening method of the invention consists of the following steps:
a1) bringing into contact the candidate compound to be tested with a mixture of :
   (1) pro-cath-D, and
   (2) the poplypeptide F4 or a substantially homologous or substantially similar amino acid sequence thereof
a2) determining the ability of said candidate compound to inhibit the binding between said pro-cath-D and F4.
The inventors have also underlined that a peptide so called "49-mer" defined by the sequence by the sequence of amino acid between 349 and 397 of LRP1 β chain as set forth in SEQ ID NO: 5.
Thus, in a preferred embodiment step a) of the screening method of the invention consists of the following steps:
a1) bringing into contact the candidate compound to be tested with a mixture of :
   (1) pro-cath-D, and
   (2) the "49 mer" polypeptide or a substantially homologous or substantially similar amino acid sequence thereof
a2) determining the ability of said candidate compound to inhibit the binding between said pro-cath-D and"49 mer".

In one embodiment the step a2) consists in generating physical values which illustrate or not the ability of said candidate compound to inhibit the interaction between said first polypeptide and said above-defined derived polypeptides of interest and comparing said values with standard physical values obtained in the same assay performed in the absence of the said candidate compound. The "physical values" that are referred to above may be of various kinds depending of the binding assay that is performed, but notably encompass light absorbance values, radioactive signals and intensity value of fluorescence signal. If after the comparison of the physical values with the standard physical values, it is determined that the said candidate compound inhibits the binding between said first polypeptide and said second polypeptide, then the candidate is positively selected at step b).

The compounds that inhibit the interaction between (i) pro-cath-D and (ii) the LRP1 β chain encompass those compounds that bind either to the pro-cath-D or to LRP1 β chain, provided that the binding of the said compounds of interest then prevents the interaction between pro-cath-D and LRP1 β chain.

### Labelled polypeptides

In one embodiment, any pro-cath-D or any LRP1 β chain derived polypeptide of the invention as defined above such as F0, F4 or "49-mer" is labelled with a detectable molecule.
According to the invention, said detectable molecule may consist of any compound or substance that is detectable by spectroscopic, photochemical, biochemical, immunochemical or chemical means. For example, useful detectable molecules include radioactive substance (including those comprising ³²P, ²⁵S, ³H, or ¹²⁵I), fluorescent dyes (including 5-bromodesosyrudin, fluorescein, acetylaminofluorene or digoxigenin), fluorescent proteins (including GFPs and YFPs), or detectable proteins or peptides (including biotin, polyhistidine tails or other antigen tags like the HA antigen, the FLAG antigen, the c-myc antigen and the DNP antigen).
According to the invention, the detectable molecule is located at, or bound to, an amino acid residue located outside the said amino acid sequence of interest, in order to minimise or prevent any artefact for the binding between said polypeptides or between the candidate compound and or any of said polypeptides.
In another particular embodiment, the polypeptides of the invention are fused with a GST tag (Glutathione S-transferase). In this embodiment, the GST moiety of the said fusion protein may be used as detectable molecule. In the said fusion protein, the GST may be located either at the N-terminal end or at the C-terminal end. The GST detectable molecule may be detected when it is subsequently brought into contact with an anti-GST antibody, including with a labelled anti-GST antibody. Anti-GST antibodies labelled with various detectable molecules are easily commercially available.
In another particular embodiment, the polypeptides of the invention are fused with a poly-histidine tag. Said poly-histidine tag usually comprises at least four consecutive hisitidine residues and generally at least six consecutive histidine residues. Such a polypeptide tag may also comprise up to 20 consecutive histidine residues. Said poly-histidine tag may be located either at the N-terminal end or at the C-terminal end. In this embodiment, the poly-histidine tag may be detected when it is subsequently brought into contact with an anti- poly-histidine antibody, including with a labelled anti- poly-histidine antibody. Anti- poly-histidine antibodies labelled with various detectable molecules are easily commercially available.
In a further embodiment, the polypeptides of the invention are fused with a protein moiety consisting of either the DNA binding domain or the activator domain of a transcription factor. Said protein moiety domain of transcription may be located either at the N-terminal end or at the C-terminal end. Such a DNA binding domain may consist of the well-known DNA binding domain of LexA protein originating form E. Coli. Moreover said activator domain of a transcription factor may consist of the activator domain of the well-known Gal4 protein originating from yeast.

### Two-hybrid assay

In one embodiment of the screening method according to the invention, the pro-cath-D and LRP1 β chain derived polypeptides, comprise a portion of a transcription factor. In said assay, the binding together of the first and second portions generates a functional transcription factor that binds to a specific regulatory DNA sequence, which in turn induces expression of a reporter DNA sequence, said expression being further detected and/or measured. A positive detection of the expression of said reporter DNA sequence means that an active transcription factor is formed, due to the binding together of said first pro-cath-D derived polypeptide and the above-defined LRP1 β chain derived polypeptides.
Usually, in a two-hybrid assay, the first and second portion of a transcription factor consist respectively of (i) the DNA binding domain of a transcription factor and (ii) the activator domain of a transcription factor. In some embodiments, the DNA binding domain and the activator domain both originate from the same naturally occurring transcription factor. In some embodiments, the DNA binding domain and the activator domain originate from distinct naturally occurring factors, while, when bound together, these two portions form an active transcription factor. The term "portion" when used herein for transcription factor, encompasses complete proteins involved in multi protein transcription factors, as well as specific functional protein domains of a complete transcription factor protein.
Therefore in one embodiment of the invention, step a) of the screening method of the invention comprises the following steps :
(1) providing a host cell expressing :
   - a first fusion polypeptide between (i) a pro-cath-D derived polypeptide and (ii) a first protein portion of transcription factor
   - a second fusion polypeptide between (i) a LRP1 β chain derived polypeptide as defined above and (ii) a second portion of a transcription factor
   said transcription factor being active on DNA target regulatory sequence when the first and second protein portion are bound together and
   said host cell also containing a nucleic acid comprising (i) a regulatory DNA sequence that may be activated by said active transcription factor and (ii) a DNA report sequence that is operatively linked to said regulatory sequence
(2) bringing said host cell provided at step 1) into contact with a candidate compound to be tested
(3) determining the expression level of said DNA reporter sequence.

Examples of LRP1 β chain derived polypeptide include, but are not limitated to F0 a F4 and "49-mer".
The expression level of said DNA reporter sequence that is determined at step (3) above is compared with the expression of said DNA reporter sequence when step (2) is omitted. A lower expression level of said DNA reporter sequence in the presence of the candidate compound means that the said candidate compound effectively inhibits the binding between the Gag derived polypeptide and the hDlg derived polypeptide and that said candidate compound may be positively selected a step b) of the screening method.
Suitable host cells include, without limitation, prokaryotic cells (such as bacteria) and eukaryotic cells (such as yeast cells, mammalian cells, insect cells, plant cells, etc.). However preferred host cell are yeast cells and more preferably a *Saccharomyces cerevisiae* cell or a *Schizosaccharomyces pombe* cell.
Similar systems of two-hybrid assays are well known in the art and therefore can be used to perform the screening method according to the invention (see. Fields et al. 1989 ; Vasavada et al. 1991 ; Fearon et al. 1992 ; Dang et al., 1991, Chien et al. 1991, US 5,283,173, US 5,667,973, US 5,468,614, US 5,525,490 and US 5,637,463). For instance, as described in these documents, the Gal4 activator domain can be used for performing the screening method according to the invention. Gal4 consists of two physically discrete modular domains, one acting as the DNA binding domain, the other one functioning as the transcription-activation domain. The yeast expression system described in the foregoing documents takes advantage of this property. The expression of a Gal1-LacZ reporter gene under the control of a Gal4-activated promoter depends on the reconstitution of Gal4 activity via protein-protein interaction. Colonies containing interacting polypeptides are detected with a chromogenic substrate for β-galactosidase. A compete kit (MATCHMAKER, TM) for identifying protein-protein interactions is commercially available from Clontech.

So in one embodiment, a first pro-cath-D derived polypeptide as above defined is fused to the DNA binding domain of Gal4 and the second LRP1 β chain derived polypeptide as above defined is fused to the activation domain of Gal4.
Examples of LRP1 β chain derived polypeptide include, but are not limitated to F0, F4 and "49-mer" as defined above in section "screening methods".
The expression of said detectable marker gene may be assessed by quantifying the amount of the corresponding specific mRNA produced. However, usually the detectable marker gene sequence encodes for detectable protein, so that the expression level of the said detectable marker gene is assessed by quantifying the amount of the corresponding protein produced. Techniques for quantifying the amount of mRNA or protein are well known in the art. For example, the detectable marker gene placed under the control of regulatory sequence may consist of the β-galactosidase as above described.

### Western blotting

In another one embodiment, step a) comprises a step of subjecting to a gel migration assay the mixture of the first pro-cath-D derived polypeptide and the second LRP1 β chain derived polypeptide as above defined, with or without the candidate compound to be tested and then measuring the binding of the said polypeptides altogether by performing a detection of the complexes formed between said polypeptides. The gel migration assay can be carried out as known by the one skilled in the art.
Therefore in one embodiment of the invention, step a) of the screening method of the invention comprises the following steps:
(1) providing a first pro-cath-D derived polypeptide and a second LRP1 β chain derived polypeptide as defined above
(2) bringing into contact the candidate compound to be tested with said polypeptides
(3) performing a gel migration assay a suitable migration substrate with said polypeptides and said candidate compound as obtained at step (2)
(4) detecting and quantifying the complexes formed between said polypeptides on the migration assay as performed at step (3).

Examples of LRP1 β chain derived polypeptide include, but are not limitated to F0, F4 and "49-mer".
The presence or the amount of the complexes formed between the polypeptides is then compared with the results obtained when the assay is performed in the absence of the candidate compound to be tested. Therefore, when no complexes between the polypeptides is detected or, alternatively when those complexes are present in a lower amount compared to the amount obtained in the absence of the candidate compound, then the candidate compound may be positively selected at step b) of the screening method.
The detection of the complexes formed between the said two polypeptides may be easily performed by staining the migration gel with a suitable dye and then determining the protein bands corresponding to the protein analysed since the complexes formed between the first and the second polypeptides possess a specific apparent molecular weight. Staining of proteins in gels may be done using the standard Coomassie brilliant blue (or PAGE blue), Amido Black, or silver stain reagents of different kinds. Suitable gels are well known in the art such as sodium dodecyl (lauryl) sulfate-polyacrylamide gel. In a general manner, western blotting assays are well known in the art and have been widely described (Rybicki et al., 1982; Towbin et al. 1979; Kurien et al. 2006).
In a particular embodiment, the protein bands corresponding to the polypeptides submitted to the gel migration assay can be detected by specific antibodies. It may be used both antibodies directed against the pro-cath-D derived polypeptides and antibodies specifically directed against the LRP1 β chain derived polypeptides according to the present invention.

In another embodiment, the said two polypeptides are labelled with a detectable antigen as above described. Therefore, the proteins bands can be detected by specific antibodies directed against said detectable antigen. Preferably, the detectable antigen conjugates to the pro-cath-D derived polypeptide is different from the antigen conjugated to the LRP1 β chain derived polypeptide. For instance, the first pro-cath-D derived polypeptide can be fused to a GST detectable antigen and the second LRP1 β chain derived polypeptide can be fused with the HA antigen. Then the protein complexes formed between the two polypeptides may be quantified and determined with antibodies directed against the GST and HA antigens respectively.

### Biosensor assays

In another embodiment, step a) included the use of an optical biosensor such as described by Edwards et al. (1997) or also by Szabo et al. (1995). This technique allows the detection of interactions between molecules in real time, without the need of labelled molecules. This technique is indeed bases on the surface plasmon resonance (SPR) phenomenon. Briefly, a first protein partner is attached to a surface (such as a carboxymethyl dextran matrix). Then the second protein partner is incubated with the previously immobilised first partner, in the presence or absence of the candidate compound to be tested. Then the binding including the binding level, or the absence of binding between said protein partner is detected. For this purpose, a light beam is directed towards the side of the surface area of the substrate that does not contain the sample to be tested and is reflected by said surface. The SPR phenomenon causes a decrease in the intensity of the reflected light with a combination of angle and wavelength. The binding of the first and second protein partner causes a change in the refraction index on the substrate surface, which change is detected as a change in the SPR signal.

### Affinity chromatography

In another one embodiment of the invention, the screening method includes the use of affinity chromatography.
Candidate compounds for use in the screening method above can also be selected by any immunoaffinity chromatography technique using any chromatographic substrate onto which (i) the first pro-cath-D derived polypeptide or (ii) the second LRP1 β chain derived polypeptide as above defined, has previously been immobilised, according to techniques well known from the one skilled in the art. Briefly, the pro-cath-D derived polypeptide or the LRP1 β chain derived polypeptide as above defined, may be attached to a column using conventional techniques including chemical coupling to a suitable column matrix such as agarose, Affi Gel®, or other matrices familiar to those of skill in the art. In some embodiment of this method, the affinity column contains chimeric proteins in which the pro-cath-D derived polypeptide or LRP1 β chain derived polypeptide as above defined, is fused to glutathion-s-transferase (GST). Then a candidate compound is brought into contact with the chromatographic substrate of the affinity column previously, simultaneously or subsequently to the other polypeptide among the said first and second polypeptide. The after washing, the chromatography substrate is eluted and the collected elution liquid is analysed by detection and/or quantification of the said later applied first or second polypeptide, so as to determine if, and/or to which extent, the candidate compound has impaired the binding between (i) first pro-cath-D derived polypeptide and (ii) the second LRP1 β chain derived polypeptide.
Examples of LRP1 β chain derived polypeptide include, but are not limitated to F0, F4, "49-mer" as defined above.

### Fluorescence assays

In another one embodiment of the screening method according to the invention, the first pro-cath-D derived polypeptide and the second LRP1 β chain derived polypeptide as above defined are labelled with a fluorescent molecule or subtrate. Therefore, the potential alteration effect of the candidate compound to be tested on the binding between the first pro-cath-D derived polypeptide and the second LRP1 β chain derived polypeptide as above defined is determined by fluorescence quantification.
Examples of LRP1 β chain derived polypeptide include, but are not limitated to F0, F4, "49-mer" as defined above.
For example, the first pro-cath-D derived polypeptide and the second LRP1 β chain derived polypeptide as above defined may be fused with auto-fluorescent polypeptides, as GFP or YFPs as above described. The first pro-cath-D derived polypeptide and the second LRP1 β chain derived polypeptide as above defined may also be labelled with fluorescent molecules that are suitable for performing fluorescence detection and/or quantification for the binding between said polypeptides using fluorescence energy transfer (FRET) assay. The first pro-cath-D derived polypeptide and the second LRP1 β chain derived polypeptide as above defined may be directly labelled with fluorescent molecules, by covalent chemical linkage with the fluorescent molecule as GFP or YFP. The first pro-cath-D derived polypeptide and the second LRP1 β chain derived polypeptide as above defined may also be indirectly labelled with fluorescent molecules, for example, by non covalent linkage between said polypeptides and said fluorescent molecule. Actually, said first pro-cath-D derived polypeptide and second LRP1 β chain derived polypeptide as above defined may be fused with a receptor or ligand and said fluorescent molecule may be fused with the corresponding ligand or receptor, so that the fluorescent molecule can non-covalently bind to said first pro-cath-D derived polypeptide and second LRP1 β chain derived polypeptide. A suitable receptor/ligand couple may be the biotin/streptavifin paired member or may be selected among an antigen/antibody paired member. For example, a polypeptide according to the invention may be fused to a poly-histidine tail and the fluorescent molecule may be fused with an antibody directed against the poly-histidine tail.
As already specified, step a) of the screening method according to the invention encompasses determination of the ability of the candidate compound to inhibit the interaction between the pro-cath-D derived polypeptide and the LRP1 β chain derived polypeptide as above defined by fluorescence assays using FRET. Thus, in a particular embodiment, the first pro-cath-D derived polypeptide as above defined is labelled with a first fluorophore substance and the second LRP1 β chain derived polypeptide is labelled with a second fluorophore substance. The first fluorophore substance may have a wavelength value that is substantially equal to the excitation wavelength value of the second fluorophore, whereby the bind of said first and second polypeptides is detected by measuring the fluorescence signal intensity emitted at the emission wavelength of the second fluorophore substance. Alternatively, the second fluorophore substance may also have an emission wavelength value of the first fluorophore, whereby the binding of said and second polypeptides is detected by measuring the fluorescence signal intensity emitted at the wavelength of the first fluorophore substance.
The fluorophores used may be of various suitable kinds, such as the well-known lanthanide chelates. These chelates have been described as having chemical stability, long-lived fluorescence (greater than 0,1 ms lifetime) after bioconjugation and significant energy-transfer in specificity bioaffinity assay. Document US 5,162,508 discloses bipyridine cryptates. Polycarboxylate chelators with TEKES type photosensitizers (EP0203047A1) and terpyridine type photosensitizers (EP0649020A1) are known. Document WO96/00901 discloses diethylenetriaminepentaacetic acid (DPTA) chelates which used carbostyril as sensitizer. Additional DPT chelates with other sensitizer and other tracer metal are known for diagnostic or imaging uses (e.g., EP0450742A1).
In a preferred embodiment, the fluorescence assay performed at step a) of the screening method consists of a Homogeneous Time Resolved Fluorescence (HTRF) assay, such as described in document WO 00/01663 or US6,740,756, the entire content of both documents being herein incorporated by reference. HTRF is a TR-FRET based technology that uses the principles of both TRF (time-resolved fluorescence) and FRET. More specifically, the one skilled in the are may use a HTRF assay based on the time-resolved amplified cryptate emission (TRACE) technology as described in Leblanc et al. (2002). The HTRF donor fluorophore is Europium Cryptate, which has the long-lived emissions of lanthanides coupled with the stability of cryptate encapsulation. XL665, a modified allophycocyanin purified from red algae, is the HTRF primary acceptor fluorophore. When these two fluorophores are brought together by a biomolecular interaction, a portion of the energy captured by the Cryptate during excitation is released through fluorescence emission at 620nm, while the remaining energy is transfered to XL665. This energy is then released by XL665 as specific fluorescence at 665 nm. Light at 665nm is emitted only through FRET with Europium. Because Europium Cryptate is always present in the assay, light at 620nm is detected even when the biomolecular interaction does not bring XL665 within close proximity.
Therefore in one embodiment, step a) of the screening method may therefore comprises the steps consisting of:
(1) bringing into contact a pre-assay sample comprising :
   - a first pro-cath-D derived polypeptide fused to a first antigen,
   - a second LRP1 β chain derived polypeptide fused to a second antigen
   - a candidate compound to be tested
(2) adding to the said pre assay sample of step (2) :
   - at least one antibody labelled with a European Cryptate which is specifically directed against the first said antigen
   - at least one antibody labelled with XL665 directed against the second said antigen
(3) illuminating the assay sample of step (2) at the excitation wavelength of the said European Cryptate
(4) detecting and/or quantifying the fluorescence signal emitted at the XL665 emission wavelength
(5) comparing the fluorescence signal obtained at step (4) to the fluorescence obtained wherein pre assay sample of step (1) is prepared in the absence of the candidate compound to be tested.
If at step (5) as above described, the intensity value of the fluorescence signal is lower than the intensity value of the fluorescence signal found when pre assay sample of step (1) is prepared in the absence of the candidate compound to be tested, then the candidate compound may be positively selected at step b) of the screening method.
Examples of LRP1 β chain derived polypeptide include, but are not limitated to F0, F4, "49-mer".
Antibodies labelled with a European Cryptate or labelled with XL665 can be directed against different antigens of interest including GST, poly-histidine tail, DNP, c-myx, HA antigen and FLAG which include. Such antibodies encompass those which are commercially available from CisBio (Bedfors, MA, USA), and notably those referred to as 61GSTKLA or 61 HISKLB respectively.

### Candidate compounds

According to a one embodiment of the invention, the candidate compound of may be selected from a library of compounds previously synthesised, or a library of compounds for which the structure is determined in a database, or from a library of compounds that have been synthesised de novo.
The candidate compound may be selected from the group consisting of (a) proteins or peptides, (b) nucleic acids and (c) organic or chemical compounds. Illustratively, libraries of pre-selected candidate nucleic acids may be obtained by performing the SELEX method as described in documents US 5,475,096 and US 5,270,163. Further illustratively, the candidate compound may be selected from the group of antibodies directed against said pro-cath-D derived polypeptide and said LRP1 β chain derived polypeptides as above described.

### In cellulo screening methods

The candidate compounds that have been positively selected at the end of any one of the embodiments of the *in vitro* screening which has been described previously in the present specification may be subjected to further selection steps in view of further assaying its antitumoral and antimetastatic properties. For this purpose, the candidate compounds that have been positively selected with the general *in vitro* screening method as above described may be further selected for their ability to inhibit the development of cancer notably cancer overexpressing cath-D and the formation of metastasis, notably fibroblast invasive growth.

Thus, another aspect of the present invention consists of a method for the *in cellulo* screening of compounds that inhibit the development of cancer and the formation of metastasis notably the fibroblast invasive growth, wherein said method comprises the steps of :
i) screening for compounds that inhibit the interaction between the pro-cath-D and the LRP1 β chain or others LRP1 β chain derived polypeptides as defined above, by performing the *in vitro* screening method as above described and
ii) screening the compounds positively selected at the end of step i) for their ability to inhibit the development of cancer and the formation of metastasis, notably by inhibiting fibroblast invasive growth.

### Pharmaceutical compositions

The inhibitor of LRP1 expression and/or the inhibitor of the interaction between pro-cath-D and LRP1 β chain may be combined with pharmaceutically acceptable excipients, and optionally sustained-release matrices, such as biodegradable polymers, to form therapeutic compositions. In the pharmaceutical compositions of the present invention for oral, sublingual, subcutaneous, intramuscular, intravenous, transdermal, local or rectal administration, the active principle, alone or in combination with another active principle, can be administered in a unit administration form, as a mixture with conventional pharmaceutical supports, to animals and human beings. Suitable unit administration forms comprise oral-route forms such as tablets, gel capsules, powders, granules and oral suspensions or solutions, sublingual and buccal administration forms, aerosols, implants, subcutaneous, transdermal, topical, intraperitoneal, intramuscular, intravenous, subdermal, transdermal, intrathecal and intranasal administration forms and rectal administration forms.
Preferably, the pharmaceutical compositions contain vehicles which are pharmaceutically acceptable for a formulation capable of being injected. These may be in particular isotonic, sterile, saline solutions (monosodium or disodium phosphate, sodium, potassium, calcium or magnesium chloride and the like or mixtures of such salts), or dry, especially freeze-dried compositions which upon addition, depending on the case, of sterilized water or physiological saline, permit the constitution of injectable solutions.
The pharmaceutical forms suitable for injectable use include sterile aqueous solutions or dispersions; formulations including sesame oil, peanut oil or aqueous propylene glycol; and sterile powders for the extemporaneous preparation of sterile injectable solutions or dispersions. In all cases, the form must be sterile and must be fluid to the extent that easy syringability exists. It must be stable under the conditions of manufacture and storage and must be preserved against the contaminating action of microorganisms, such as bacteria and fungi.
Solutions comprising compounds of the invention as free base or pharmacologically acceptable salts can be prepared in water suitably mixed with a surfactant, such as hydroxypropylcellulose. Dispersions can also be prepared in glycerol, liquid polyethylene glycols, and mixtures thereof and in oils. Under ordinary conditions of storage and use, these preparations contain a preservative to prevent the growth of microorganisms.
The inhibitor of LRP1 expression and/or interaction between pro-cath-D and LRP1 β chain of the invention can be formulated into a composition in a neutral or salt form. Pharmaceutically acceptable salts include the acid addition salts (formed with the free amino groups of the protein) and which are formed with inorganic acids such as, for example, hydrochloric or phosphoric acids, or such organic acids as acetic, oxalic, tartaric, mandelic, and the like. Salts formed with the free carboxyl groups can also be derived from inorganic bases such as, for example, sodium, potassium, ammonium, calcium, or ferric hydroxides, and such organic bases as isopropylamine, trimethylamine, histidine, procaine and the like.
The carrier can also be a solvent or dispersion medium containing, for example, water, ethanol, polyol (for example, glycerol, propylene glycol, and liquid polyethylene glycol, and the like), suitable mixtures thereof, and vegetables oils. The proper fluidity can be maintained, for example, by the use of a coating, such as lecithin, by the maintenance of the required particle size in the case of dispersion and by the use of surfactants. The prevention of the action of microorganisms can be brought about by various antibacterial and antifungal agents, for example, parabens, chlorobutanol, phenol, sorbic acid, thimerosal, and the like. In many cases, it will be preferable to include isotonic agents, for example, sugars or sodium chloride. Prolonged absorption of the injectable compositions can be brought about by the use in the compositions of agents delaying absorption, for example, aluminium monostearate and gelatin.
Sterile injectable solutions are prepared by incorporating the active polypeptides in the required amount in the appropriate solvent with various of the other ingredients enumerated above, as required, followed by filtered sterilization. Generally, dispersions are prepared by incorporating the various sterilized active ingredients into a sterile vehicle which contains the basic dispersion medium and the required other ingredients from those enumerated above. In the case of sterile powders for the preparation of sterile injectable solutions, the preferred methods of preparation are vacuum-drying and freeze-drying techniques which yield a powder of the active ingredient plus any additional desired ingredient from a previously sterile-filtered solution thereof.
Upon formulation, solutions will be administered in a manner compatible with the dosage formulation and in such amount as is therapeutically effective. The formulations are easily administered in a variety of dosage forms, such as the type of injectable solutions described above, but drug release capsules and the like can also be employed.
For parenteral administration in an aqueous solution, for example, the solution should be suitably buffered if necessary and the liquid diluent first rendered isotonic with sufficient saline or glucose. These particular aqueous solutions are especially suitable for intravenous, intramuscular, subcutaneous and intraperitoneal administration. In this connection, sterile aqueous media which can be employed will be known to those of skill in the art in light of the present disclosure. For example, one dosage could be dissolved in 1 ml of isotonic NaCl solution and either added to 1000 ml of hypodermoclysis fluid or injected at the proposed site of infusion. Some variation in dosage will necessarily occur depending on the condition of the subject being treated. The person responsible for administration will, in any event, determine the appropriate dose for the individual subject.
The inhibitor of LRP1 expression and/or interaction between pro-cath-D and LRP1 β chain of the invention may be formulated within a therapeutic mixture to comprise about 0.0001 to 1.0 milligrams, or about 0.001 to 0.1 milligrams, or about 0.1 to 1.0 or even about 10 milligrams per dose or so. Multiple doses can also be administered.
In addition to the compounds of the invention formulated for parenteral administration, such as intravenous or intramuscular injection, other pharmaceutically acceptable forms include, e.g. tablets or other solids for oral administration; liposomal formulations; time release capsules; and any other form currently used.

### Diagnostic methods

The present invention relates to a method for diagnosing a cancer secreting cath-D or Alzheimer's disease in a patient comprising detecting the presence of a fragment of LRP1 in a biological sample obtained from said patient.
Preferably said fragment of LRP1 is LRP1 β chain ecto-domain as set forth in SEQ ID NO:2 or LRP1 α chain or fragments thereof.

Once the biological sample from the patient is prepared, the fragment of LRP1 may be detected and the concentration measured by any known method in the art.
For example, the concentration of LRP1 β chain ecto-domain may be measured by using standard electrophoretic and immunodiagnostic techniques, including immunoassays such as competition, direct reaction, or sandwich type assays. Such assays include, but are not limited to, Western blots; agglutination tests; enzyme-labeled and mediated immunoassays, such as ELISAs; biotin/avidin type assays; radioimmunoassays; immunoelectrophoresis; immunoprecipitation, high performance liquid chromatography (HPLC), size exclusion chromatography, solid-phase affinity, etc.
In a particular embodiment, such methods comprise contacting the biological sample with a binding partner capable of selectively interacting with LRP1 β chain ecto-domain present in the biological sample.
The binding partner may be generally an antibody that may be polyclonal or monoclonal, preferably monoclonal. Antibodies directed against LRP1 β chain ecto-domain can be prepared and isolated according to methods and techniques described above.
The binding partners of the invention such as antibodies or aptamers, may be labelled with a detectable molecule or substance, such as a fluorescent molecule, a radioactive molecule or any others labels known in the art. Labels are known in the art that generally provide (either directly or indirectly) a signal.
As used herein, the term "labeled", with regard to the antibody, is intended to encompass direct labeling of the antibody or aptamer by coupling (i.e., physically linking) a detectable substance, such as a radioactive agent or a fluorophore (e.g. fluorescein isothiocyanate (FITC) or phycoerythrin (PE) or Indocyanine (Cy5)) to the antibody or aptamer, as well as indirect labeling of the probe or antibody by reactivity with a detectable substance. An antibody or aptamer of the invention may be labelled with a radioactive molecule by any method known to the art. For example radioactive molecules include but are not limited radioactive atom for scintigraphic studies such as I¹²³, I¹²⁴, In¹¹¹, Re¹⁸⁶, Re¹⁸⁸.
The aforementioned assays generally involve the bounding of the binding partner (ie. Antibody or aptamer) in a solid support. Solid supports which can be used in the practice of the invention include substrates such as nitrocellulose (e. g., in membrane or microtiter well form); polyvinylchloride (e. g., sheets or microtiter wells); polystyrene latex (e.g., beads or microtiter plates); polyvinylidine fluoride; diazotized paper; nylon membranes; activated beads, magnetically responsive beads, and the like.
More particularly, an ELISA method can be used, wherein the wells of a microtiter plate are coated with a set of antibodies against LRP1 β chain ecto-domain. A biological sample containing or suspected of containing LRP1 β chain ecto-domain is then added to the coated wells. After a period of incubation sufficient to allow the formation of antibody-antigen complexes, the plate (s) can be washed to remove unbound moieties and a detectably labeled secondary binding molecule added. The secondary binding molecule is allowed to react with any captured sample marker protein, the plate washed and the presence of the secondary binding molecule detected using methods well known in the art.
By way of example, an antibody useful in the diagnostic methods according to the present invention consists in the monoclonal anti-α2 macroglobulin receptor (# 3501, American Diagnostica Inc).
Measuring concentration of LRP1 fragments, LRP1 β chain ecto-domain in particular (with or without immunoassay-based methods) may also include separation of the proteins: centrifugation based on the protein's molecular weight; electrophoresis based on mass and charge; HPLC based on hydrophobicity; size exclusion chromatography based on size; and solid-phase affinity based on the protein's affinity for the particular solid-phase that is use. Once separated, the LRP1 fragment, LRP1 β chain ecto-domain in particular, may be identified based on the known "separation profile," e. g., retention time, for that protein and measured using standard techniques.

Alternatively, the separated proteins may be detected and measured by, for example, a mass spectrometer.
Alternatively, the method of the invention further may comprise a step consisting in comparing the concentration of the LRP1 fragment (e.g. LRP1 β chain ecto-domain) measured as above described with a predetermined value. Therefore, said comparison can be indicative of the severity or morbidity of organ dysfunction in said patient.
A further object of the invention relates to a kit for diagnosing a cancer secreting cath-D or Alzheimer's disease in a patient, comprising means for detecting the presence of the LRP1 β chain ecto-domain or of LRP1 α chain or of fragments thereof. The kit may include an antibody, or a set of antibodies as above described. In a particular embodiment, the antibody or set of antibodies are labelled as above described. The kit may also contain other suitably packaged reagents and materials needed for the particular hybridization protocol, including solid-phase matrices, if applicable, and standards. The kit may also contain means for the detection of other known markers of cancer secreting cath-D or Alzheimer's disease.
Finally, the method of the invention may be applied for monitoring the therapeutic outcome of a patient affected with a cancer secreting cath-D or Alzheimer's disease.

The invention will further be illustrated in view of the following figures and examples.

### LEGEND TO FIGURES

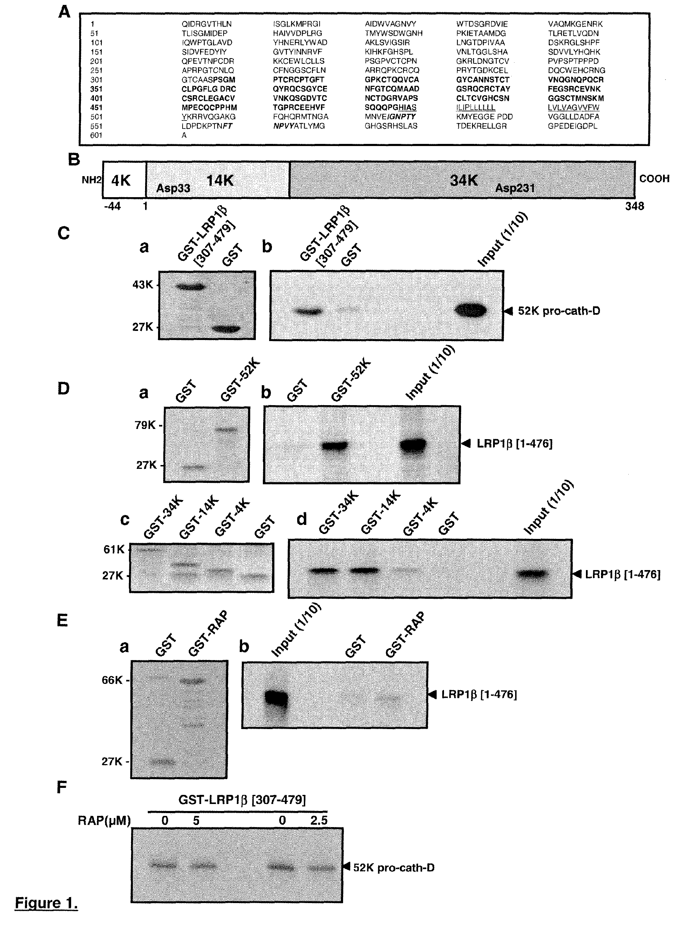

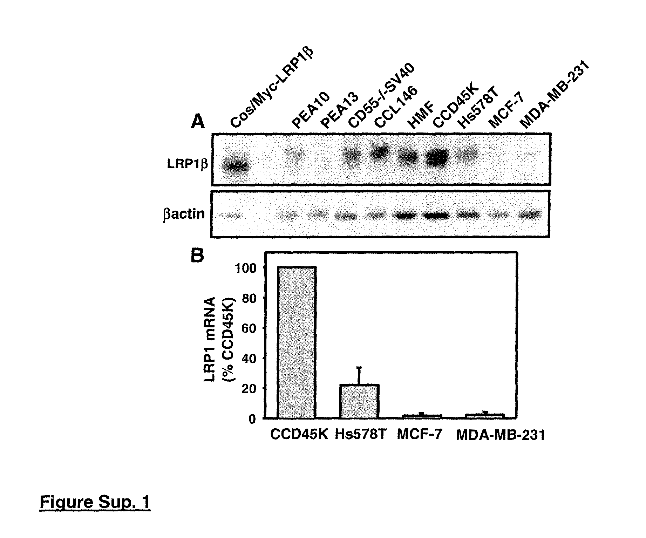

### Figure 1. Human cath-D binds to the extracellular domain of the β chain of LRP1 in vitro in a RAP insensitive manner.

**(A) Sequence of the LRP1** β **chain interacting with cath-D.** LRP1 is synthesized as a precursor of 4544 amino acids that is cleaved into an α chain and a β chain. The sequence of the β chain is shown and amino acids are numbered starting at the first residue of the β chain. Residues 307-479, which form the cath-D binding site, are shown in bold. The trans-membrane sequence is underlined, and the two NPXY motifs are in italics.
**(B) Schematic representation of the human cath-D.** Location of 4 kDa cath-D pro-fragment, 14 kDa light and 34 kDa heavy mature chains are indicated. Intermediate 48 kDa form (not shown) corresponds to non-cleaved 14 + 34 kDa chains. 1 corresponds to the first amino acid of the mature cath-D (Faust et al., 1985). The position of the catalytic aspartic acids is shown.
**(C) Binding of pro-cath-D to LRP1** β **(307-479).** Radio-labelled pro-cath-D, produced by *in vitro* translation, was incubated with glutathione-Sepharose beads containing GST-LRP1 β (307-479) or GST. GST proteins bound to beads were stained by Coomassie (panel a) and bound pro-cath-D was detected by autoradiography (panel b). The lysate used for the binding reaction (input) is shown for comparison. Molecular mass is shown in K (kDa).
**(D) Binding of the full length extracellular domain of the** β **chain of LRP1** to cath-D. Radio-labelled full-length extracellular domain of LRP1β (1-476) was incubated with beads containing GST-cath-D/52K, GST-cath-D/34K, GST-cath-D/14K, GST-cath-D/4K pro-domain, or GST. GST proteins stained by Coomassie are shown in panels a and c and bound LRP1β (1-476) in panels b and d.
**(E) Binding of the extracellular domain of the** β **chain of LRP1 to RAP.** Radio-labelled full-length extracellular domain of LRP1β (1-476) was incubated with beads containing GST-RAP or GST. GST proteins stained by Coomassie are shown in panel a and bound LRP1β (1-476) in panel b.
**(F) Effect of RAP for the binding of pro-cath-D to LRP1**β **(307-479).** Radio-labelled pro-cath-D was incubated with beads containing GST-LRP1β (307-479) in the presence of increasing concentrations of RAP and bound pro-cath-D was analysed.

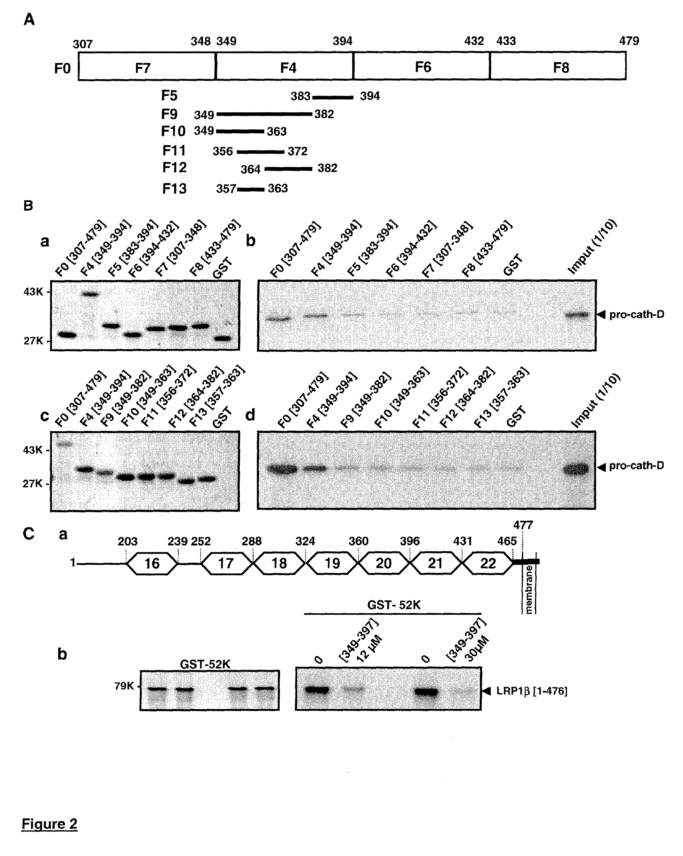

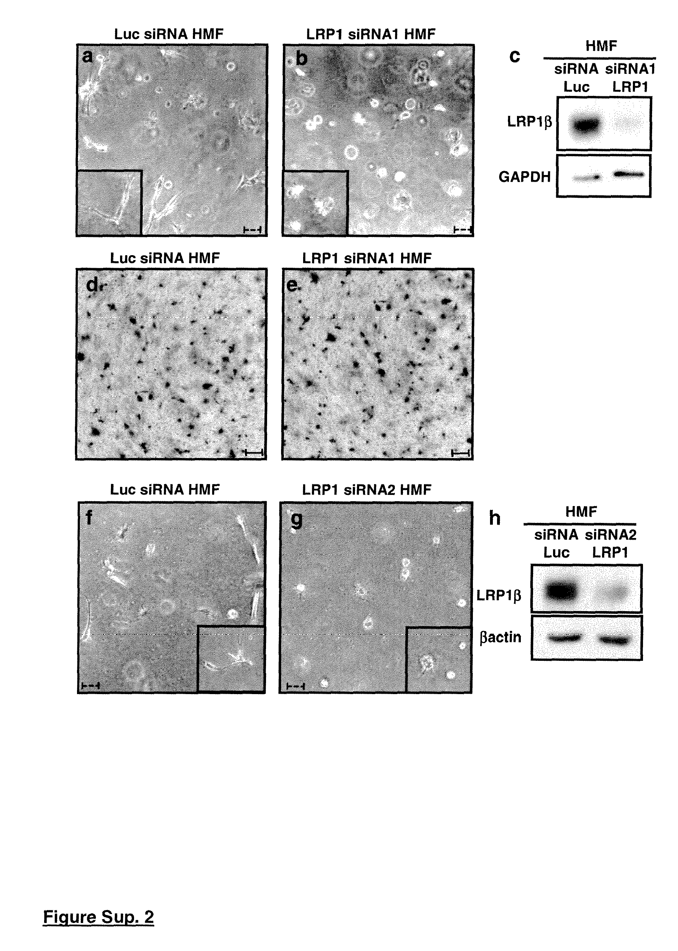

### Figure 2. Mapping of the minimal cath-D binding region on LRP1β.

**(A) Schematic representation of GST-LRP1**β **fragments.** Shorter GST-LRP1β fragments (F4, F6-F8) were derived from GST-LRP1β (307-479) (F0). The F4 (349-394) GST-LRP1β fragment was then sub-divided into shorter fragments (F5, F9-13).
**(B) Binding of pro-cath-D to GST-LRP1**β **fragments.** Radio-labelled pro-cath-D was incubated with beads containing GST-LRP1β fragments or GST. GST proteins bound to beads were stained by Coomassie (panels a, c) and bound pro-cath-D was detected by autoradiography (panels b, d).
**(C) Effect of the (349-397) LRP1 peptide for the binding of the extracellular domain of LRP1**β **(1-476) to pro-cath-D.** Schematic representation and numbering of the EGF-like repeats 16 to 22 present in the extracellular domain of LRP1β is shown in panel a. Radio-labelled extracellular domain of LRP1β (1-476) was incubated with beads containing GST-cath-D/52K in the absence or presence of increasing concentration of the (349-397) LRP1 peptide (panel b). GST proteins stained by Coomassie are shown in left panel and bound LRP1β (1-476) in right panel .

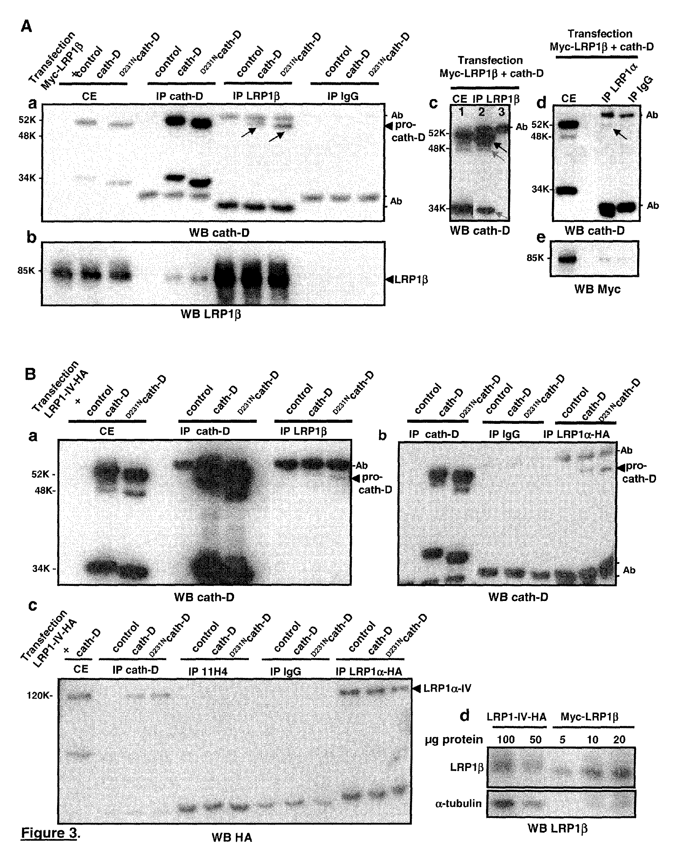

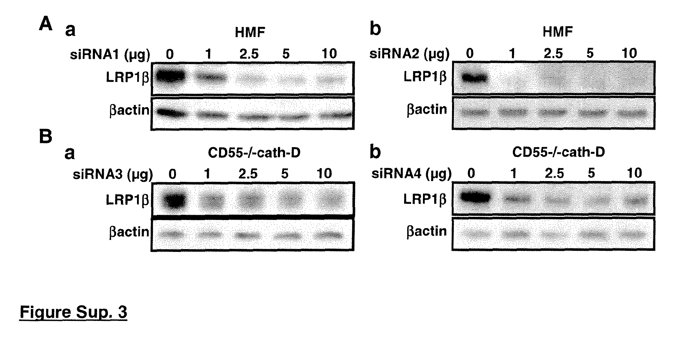

### Figure 3. Cath-D and LRP1 associate in cells.

**(A) Co-transfected cath-D and LRP1**β **associate in cells.** COS cells transiently expressing Myc-LRP1β in the presence or absence of cath-D or ^{D231N}cath-D were lysed in PLC-lysis buffer. Cath-D, LRP1β, LRP1 α and non-immune IgG control immunoprecipitates (IP) were analyzed by anti-cath-D (panels a, c and d), anti-LRP1β immunoblotting (panel b) and anti-Myc immunoblotting (panel e). A longer exposure of anti-cath-D immunoblotting is shown in panel c. Cell extracts (CE) were analyzed for cath-D expression (panels a, c and d), for LRP1 β chain expression (panel b) and for Myc- LRP1 β chain expression in panel e. In panel c, lane 1 corresponds to CE, lane 2 to IP anti-LRP1β and lane 3 to the antibody loaded alone. Black arrows show co-immunoprecipitated cath-D. Ab, antibody.
**(B) Co-transfected cath-D and LRP1-IV-HA mini-receptor associate in cells.** COS cells transiently expressing LRP1-IV-HA vector in the presence or absence of cath-D or ^{D231N}cath-D were lysed in PLC lysis buffer. Cath-D, LRP1β, LRP1α-HA and non-immune IgG control immunoprecipitates were analyzed by anti-cath-D (panels a and b) and anti-HA immunoblotting (panel c). Whole cell lysates (CE) were analyzed for cath-D (panel a) or LRP1α-IV (panel c) expression. Black arrows show co-immunoprecipitated pro-cath-D. Comparison of the expression levels of transfected Myc-LRP1β and LRP1-IV-HA is shown in panel d. Increasing amounts of protein from COS cells transiently expressing Myc-LRP1β (5, 10 and 20 µg) or LRP1-IV-HA (50 and 100 µg) were analysed for LRP1β expression by immunoblotting. α-tubulin was used as loading control.

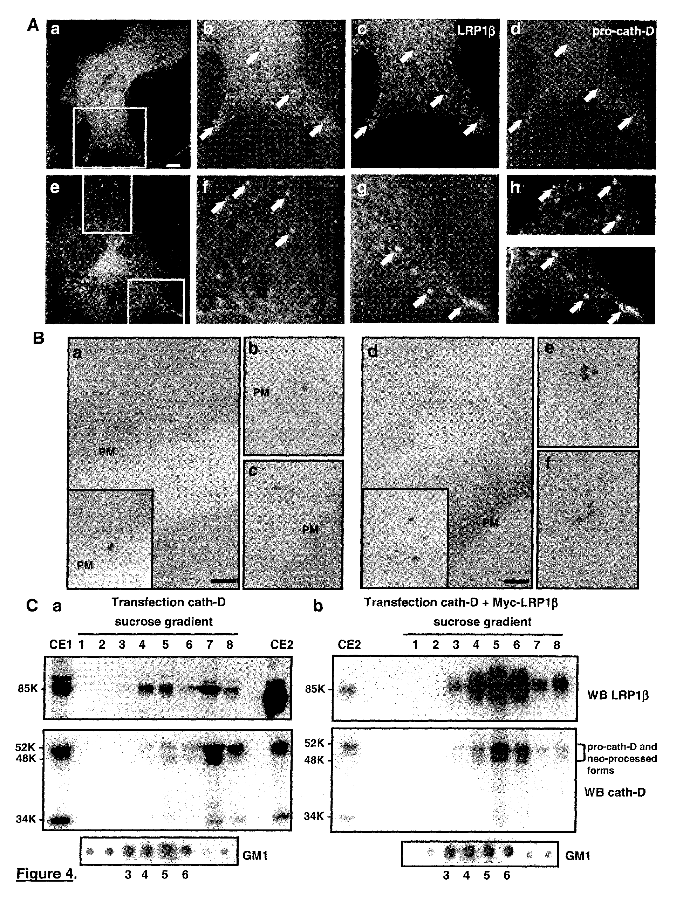

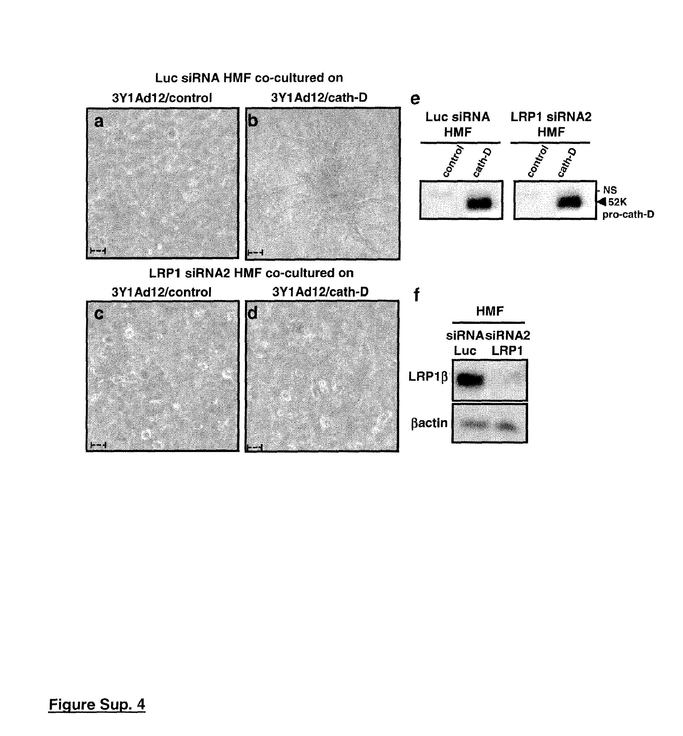

### Figure 4. Pro-cath-D and LRP1 co-distribute in cells.

**(A) Co-transfected pro-cath-D and LRP1** β **chain co-localize partially in punctuate structures.** COS cells transiently co-expressing cath-D and Myc-LRP1β were stained with a monoclonal anti-pro-cath-D antibody (red) and a polyclonal anti-LRP1β antibody (green) (panel s a-d). The boxed area in the merged image (panel a) is shown at a higher magnification (x 2.8) in panel b. Panel c shows LRP1β staining and panel d shows pro-cath-D staining. COS cells transiently co-expressing cath-D and Myc-LRP1β in the presence of M6P (10 mM) were double stained with anti-pro-cath-D antibody (red) and anti-LRP1β antibody (green) (panels e-i). The two boxed areas in the merged image (panel e) are shown at higher magnification in panels f and g. Panels h and i, that correspond respectively to panels f and g, represent co-localization analysis with the Lasersharp Software (Manders et al., 1993). Arrows indicate co-localization of pro-cath-D and LRP1β. *Bars,* 5 µm.
**(B) Immunogold-labelled pro-cath-D and LRP1** β **chain co-localize at the cell surface and in vesicular-like structures**. COS cells transiently co-expressing cath-D and Myc-LRP1β were labelled with a monoclonal anti-pro-cath-D antibody conjugated to 6 nm gold particles and with a polyclonal anti-LRP1 β chain antibody conjugated to with 15 nm gold particles. Panels a-b illustrate co-localization at the plasma membrane. Panel c evidences clusters of pro-cath-D and LRP1 close to the plasma membrane. Panel d illustrates co-localization in vesicular-like structures. Panels e and f show co-localization of several 15 nm immunogold particles targeting LRP1β with 6 nm immunogold particles targeting pro-cath-D. PM = plasma membrane. *Bars,* 100 nm.
**(C) Transfected cath-D is found in rafts and co-transfection with LRP1 β chain enhances its presence in these micro-domains.** COS cells transiently expressing cath-D (panel a) or cath-D + Myc-LRP1β (panel b) were subjected to detergent solubilization and sucrose density ultracentrifugation. After centrifugation, 8 fractions collected from the top of the gradient were analyzed by SDS-PAGE and immunoblotting for LRP1 β chain (top panels) or for cath-D (medium panels). Whole cell lysates (100 µg) from cath-D expressing (CE1) and from cath-D + Myc-LRP1β co-expressing COS (CE2) were analyzed in parallel. CE2 was loaded on both gels (panels a and b) as an internal control for cath-D or LRP1β enrichment relative to film exposure. Bracket indicates pro-cath-D and its neo-processed forms. An aliquot of each fraction was dot-blotted onto a nitrocellulose membrane to detect ganglioside GM1 with horse peroxidase-conjugated cholera toxin B subunit (bottom panels).

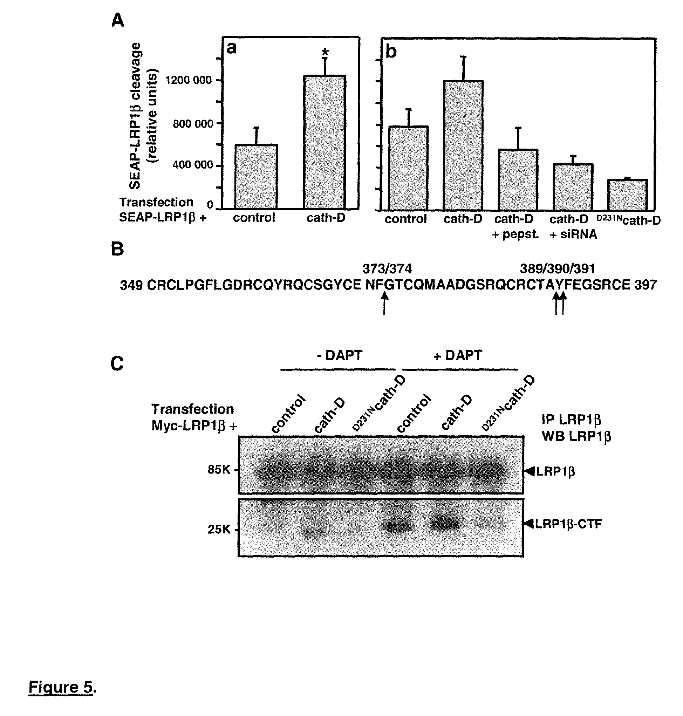

### Figure 5. Cath-D triggers LRP1 RIP.

**(A) Shedding of LRP1** β **chain ectodomain.** SEAP activity was quantified in the medium of COS cells transiently transfected with SEAP-LRP1β and β-galactosidase vectors in the absence or presence of cath-D vector (panel a). The mean ± SD of 4 independent determinations with both transfections and measurements carried in duplicate is shown *, p<0.0025 *versus* control (Student's t-test). SEAP activity was also quantified in the medium of COS cells transiently transfected with SEAP-LRP1β and β-galactosidase vectors in the presence of cath-D vector + pepstatin (100 µM), cath-D vector + cath-D siRNA, and catalytically inactive ^{D231N}cath-D vector (panel b). A representative experiment from a transfection performed in duplicate is shown. Similar results were obtained in another independent experiment.
**(B) Cleavage of the LRP1 [349-397] peptide by auto-activated pro-cath-D**. Pro-cath-D (500 ng), auto-activated at pH 3.5 for 30 min, was incubated for 2 h with 50 µM of LRP1 [349-397] peptide at pH 3.5. Cleaved peptides were resolved by reverse-phase HPLC and subjected to NH2-terminal sequence analysis. Arrows indicate the 3 cleavage sites.
**(C) Production of LRP1** β **carboxyl-terminal fragment.** COS cells, transfected with Myc-LRP1β in the absence or presence of wild-type or D231 N cath-D, were treated with DAPT (1 µM) or DMSO for 24 h. Cells were lysed and the full-length Myc-LRP1β and its carboxy-terminal fragment (LRP1β-CTF) were isolated by immunoprecipitation and visualized by immunoblotting with the monoclonal antibody 11H4.
*, p<0.025 *versus* Luc siRNA HMF cells; **, p<0.0025 *versus* Luc siRNA HMF cells (Student's *t*-test).

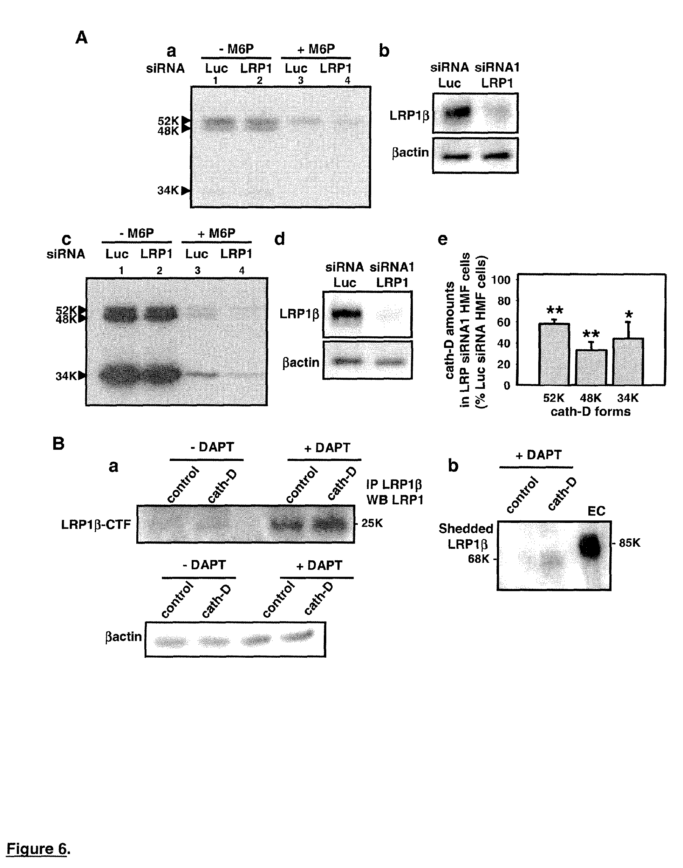

### Figure 6. Cath-D interacts with LRP1 in HMF fibroblasts.

**(A) Pro-cath-D is internalized in part by LRP1.** Conditioned media containing labelled pro-cath-D was produced by incubating cath-D expressing 3Y1-Ad12 cells with [S³⁵]methionine and [S³⁵]cysteine for 24 h. HMF fibroblasts were transiently transfected with either Luc siRNA or LRP1 siRNA1 and endocytosis of labelled pro-cath-D was performed 48 h post-transfection. For endocytosis, siRNAs-transfected HMF fibroblasts were incubated for 3 h (panel a) or for 18h (panel c) with conditioned medium containing labelled pro-cath-D in the absence or presence of 10 mM M6P. Cell lysates containing endocytosed labelled cath-D were analyzed by SDS-PAGE after immunoprecipitation with M1G8 anti-cath-D antibody (panels a and c). LRP1β expression was monitored in HMF fibroblasts by immunoblotting 48 h post-transfection with either Luc or LRP1 siRNAs (panels b and d). ßactin was used as a loading control. Quantitation of 52, 48 and 34 kDa cath-D internalized by LRP1 siRNA1 HMF cells relative to that internalized by Luc siRNA HMF cells in the presence of M6P after 16 h of endocytosis is shown in panel e (mean ± SD of 3 independent experiments).
**(B) Production of LRP1**β**-CTF and shedding of LRP1**β **in response to cath-D.** COS cells were transfected with control or cath-D vectors and 48 h post-transfection, conditioned media (control or containing 12 nM of pro-cath-D) were added for 24 h on HMF cells in the absence or presence of DAPT (1 µM). Cells (4 mg protein) were lysed and the 25 kDa LRP1β carboxyl-terminal fragment (LRP1β-CTF) was isolated and visualized by immunoprecipitation and blotting with a monoclonal antibody 11 H4 (panel a, top panel). ßactin (100 µg of cell extract) was used as a loading control for immunoprecipitation (panel a, bottom panel). Similar results were observed in 4 independent experiments. LRP1β shedded in the media of HMF fibroblasts after 24 h in the presence of DAPT (1 µM) was detected by immmunoblotting with the monoclonal antibody #3501 directed against the extracellular LRP1 β chain (panel b). A cell extract (CE; 100 µg) of HMF cells was loaded in order to detect the full length 85 kDa LRP1β.

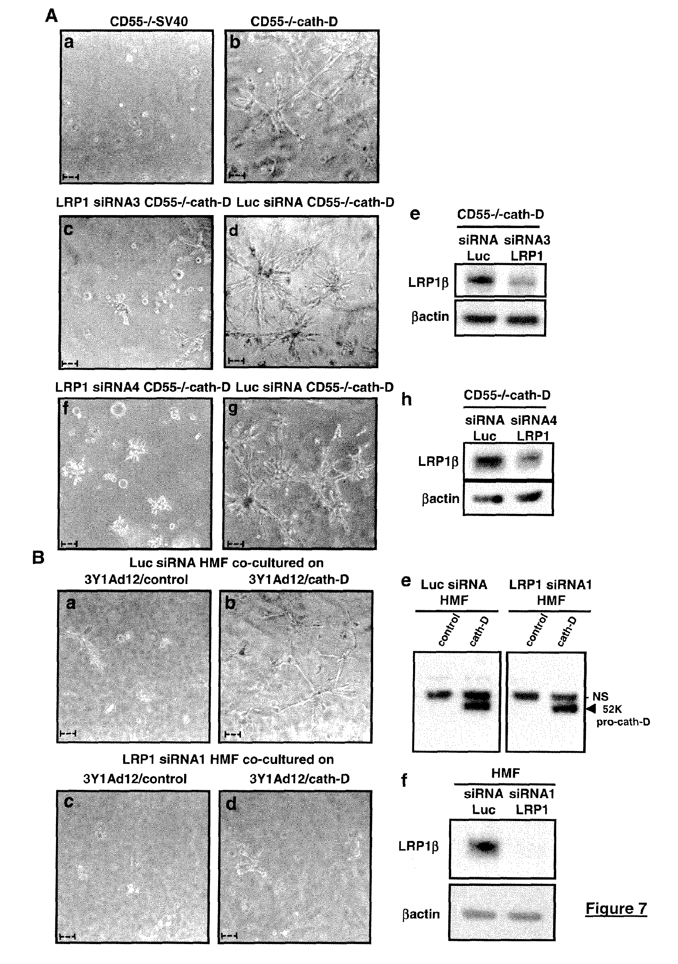

### Figure 7. cath-D promotes fibroblast invasive growth in a LRP1-dependent paracrine manner.

**(A) Cath-D promotes fibroblast invasive growth only in the presence of LRP1**. Cath-D-deficient CD55-/-SV40 (panel a) and cath-D-transfected CD55-/-cath-D (panels b-g) were embedded in Matrigel. CD55-/-cath-D cells transfected with either LRP1 siRNA3 (panel c), LRP1 siRNA4 (panel f), or Luc siRNA (panels d and g) were embedded in Matrigel 24 h post-transfection. Phase contrast optical photomicrographs after 4 days of culture are shown. Data from one representative experiment out of 2 is shown. *Bars,* 75 µm. CD55-/-cath-D cells were transfected with either Luc siRNA, LRP1 siRNA3 or LRP1 siRNA4 and LRP1β expression was monitored 24 h post-transfection (panels e and h). Equal amount of cell lysate (100 µg) was analyzed for LRP1β and β̃actin expressions by immunoblotting.
**(B) Inhibition of LRP1 in HMF fibroblasts prevents cath-D invasive growth stimulation.** HMF fibroblasts transfected with either Luc siRNA (panels a-b) or LRP1 siRNA1 (panels c, d) were embedded 48 h post-transfection in the presence of a bottom layer of 3Y1-Ad12 cancer cell lines secreting either no cath-D (control, panels a and c) or human cath-D (cath-D, panels b and d). Phase contrast optical photomicrographs of HMF fibroblasts after 3 days of co-culture are shown in panels a-d. Data from one representative experiment out of 3 is shown. *Bars* (---, 75 µm). Pro-cath-D secretion was analyzed by immunoblotting after 3 days of co-culture of either Luc siRNA (panel e, left panel) or LRP1 siRNA1 (panel e, right panel) HMF fibroblasts with 3Y1Ad12/control or 3Y1Ad12/cath-D cells. NS = non specific contaminant protein. HMF cells were transfected with either Luc siRNA or LRP1 siRNA1 and LRP1β expression was monitored 48 h post-transfection before the co-culture assays (panel f).

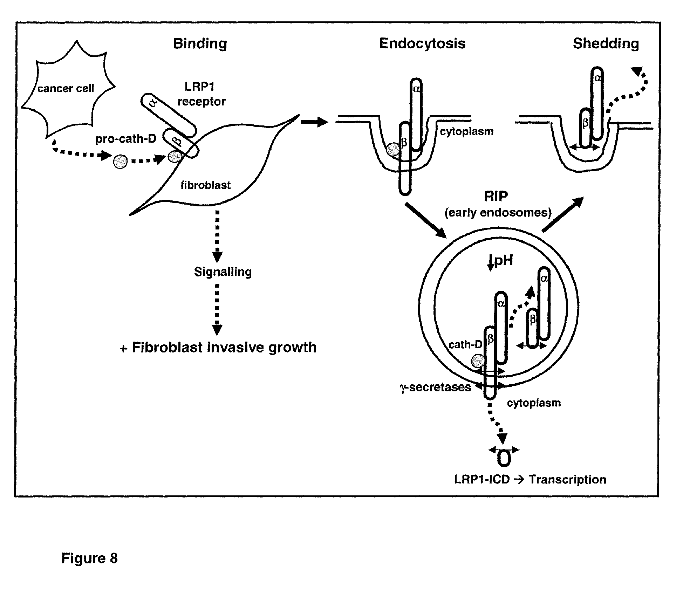

### Figure 8. Model of cath-D action through its interaction with LRP1 in fibroblast.

Pro-cath-D secreted by cancer cells binds to residues 349-394 in the extracellular domain of the β chain of LRP1, possibly in lipid rafts, in fibroblasts. Upon binding, pro-cath-D is internalized and, due to the acidification within the early endosomes, is processed. Activated cath-D cleaves LRP1 within the (349-394) binding domain. Shed LRP1 ectodomain is released in the serum through recycling vesicules. Ectodomain shedding results in the production of a 25 kDa membrane-bound fragment (LRP1β-CTF), that is further cleaved by γ-secretases, leadind to the production of LRP1-ICD, that may affect gene transcription. Since mutated ^{D231N}cath-D binds to LRP1 and does not trigger LRP1 RIP, we propose that cath-D binding to LRP1 may also lead to the activation of other unknown intracellular signalling pathways that elicit fibroblast invasive growth.

### Figure Sup. 1. Expression of LRP1 in fibroblasts and breast cancer cells.

**(A) Protein expression of LRP1** β **chain.** Whole cell extracts were prepared using PLC lysis buffer and subjected to SDS-PAGE and immunoblotting with the anti-LRP1β 11H4 hybridoma. Myc-LRP1β transfected COS cells were used as a positive control and LRP1-deficient PEA13 as a negative control. ßactin expression was used as a loading control.
**(B) RNA expression of LRP1.** mRNAs were quantified by real-time quantitative RT-PCR. Mean ± SD of 3 independent experiments is shown.

**Figure Sup. 2. LRP1 expression is required for fibroblastic morphology in three-dimensional matrices.** HMF fibroblasts transfected with either Luc siRNA (panels a, d, f), LRP1 siRNA1 (panels b and e) or LRP1 siRNA2 (panel g) were seeded in Matrigel 48 h post-transfection. Phase contrast images taken after 3 days of culture are shown (panels a, b, f, g). p-nitrotetrazolium violet cell staining after 5 day of culture is also given (panels d and e). Data from one representative experiment out of 3 is shown. *Bars* (---, 75 µm; -, 750 µm). Cells transiently expressing Luc siRNA, LRP1 siRNA1 or LRP1 siRNA2 were analyzed by immunoblotting 48 h post-transfection (panels c, h).

### Figure Sup. 3. Dose-responses of LRP1 siRNAs in fibroblasts.

**(A) Dose-responses in HMF fibroblasts.** Cells were transiently transfected with increasing concentrations of siRNA1 (panel a) or siRNA2 (panel b) for 48 h. LRP1β expression was analysed by immunoblotting using the anti-LRP1β 11H4 hybridoma. ßactin expression was used as a loading control. Dose-response of Luc siRNA presented unchanged LRP1β level (data not shown).
**(B) Dose-responses in CD55-/-cath-D fibroblasts.** Cells were transiently transfected with increasing concentrations of siRNA3 (panel a) or siRNA4 (panel b) for 48 h. LRP1β expression was analyzed as described in A. Dose-response of Luc siRNA presented unchanged LRP1β level (data not shown).

**Figure Sup. 4. Inhibition of LRP1 in HMF fibroblasts prevents cath-D invasive growth stimulation.** HMF fibroblasts transfected with either Luc siRNA (panels a, b) or LRP1 siRNA2 (panels c, d) were embedded 48 h post-transfection in the presence of a bottom layer of 3Y1-Ad12 cancer cell lines secreting either no cath-D (control, panels a and c) or human cath-D (cath-D, panels b and d). Phase contrast optical photomicrographs of HMF fibroblasts after 3 days of co-culture are shown in panels a-d. *Bars* (---, 75 µm). Pro-cath-D secretion was analyzed after 3 days of co-culture of either Luc (panel e, left panel) or LRP1 (panel e, right panel) siRNA HMF fibroblasts with 3Y1Ad12/control or 3Y1Ad12/cath-D cells by immunoblotting. NS = non specific contaminant protein. HMF cells were transfected with either Luc siRNA or LRP1 siRNA2 and LRP1β expression was monitored 48 h post-transfection before the co-culture assays (panel f).

### EXAMPLES:

### MATERIALS AND METHODS

**Materials.** Human mammary fibroblasts (HMF), kindly provided by Dr Jacques Piette (CRLC Val d'Aurelle, Montpellier, France), were obtained from reduction mammoplasty tissues from a patient without cancer. Cath-D-deficient CD55-/- immortalized mouse fibroblasts were kindly provided by Dr Christoph Peters (University of Freiburg, Germany) and Dr Paul Saftig (Christian-Albrechts-University, Germany). CD55-/-SV40 and CD55-/-cath-D cell lines were obtained by cath-D stable transfection (Laurent-Matha et al, 2005). PEA13 and PEA10 mouse fibroblasts were purchased from ATCC. All cell lines were cultured in DEM medium with 10% fetal calf serum (FCS, GibcoBRL). The hybridoma 11H4 purchased from ATCC was grown in DEM medium supplemented with 10% FCS and 25 mM L-glutamine. Polyclonal anti-human LRP1β-chain antiserum was generated by using a synthetic polypeptide corresponding to the C-terminal 13 amino acids of LRP1 (Zhang et al., 2004). The monoclonal antibody # 3501 directed against the beta chain of LRP1 (aa 4291-4344) was purchased from American Diagnostica Incorporation. The anti-human cath-D monoclonal antibody (#610800, BD Biosciences) used for immuno-blotting recognizes 52, 48 and 34 kDa cath-D forms. The anti-human cath-D M1G8 monoclonal antibody used for immunoprecipitation recognizes 52, 48 and 34 kDa cath-D forms. The anti-human cath-D monoclonal antibody M2E8 used for immuno-fluorescence interacts specifically with 52 kDa pro-cath-D. Anti-HA monoclonal antibody (12CA5 clone) was purchased from Roche, anti-GAPDH monoclonal antibody from Santa Cruz Biotechnology and anti-ßactin polyclonal antibody (#A2066) from Sigma. DAPT and pepstatin A were purchased from Sigma. The 49-mer (349-397) LRP1 peptide was synthetized by GeneCust.

**Plasmids.** pcDNA3-Myc-tagged LRP1β chain, LRP1-IV-HA mini-receptor and SEAP-LRP1β were described elsewhere (Barnes et al., 2003 ; Zhang et al., 2004 ; von Arnim et al., 2005). The SEAP-LRP1β was used instead of full-length LRP1 because of its functional similarity to and better expression than full-length LRP1 as previously described (Von Arnim et al., 2005). pcDNA3.1(-)cath-D and pSG5-neo-cath-D expression plasmids were previously described (Glondu et al., 2001; Laurent-Matha et al., 2005). pcDNA3.1(+)Myc-tagged LRP1β chain was generated by inserting cDNA encoding LRP1β obtained by *Kpn*I and *Xba*I digestion of pcDNA3-Myc-tagged LRP1β into pcDNA3.1(+) previously digested by *Kpn*I and *Xba*I. pcDNA3.1(+)LRP1β extracellular domain (1-476) expression plasmid was created by inserting the PCR-amplified cDNA encoding LRP1β (1-476) from pcDNA3.1(+)Myc-tagged LRP1β into pcDNA3.1(+) previously digested by *Nhe*I and *Xba*I. pGEX-4T-1 LRP1β (307-479) was generated by inserting PCR-amplified cDNA encoding LRP1β (307-479) from pYESTrp2-LRP1β (307-479) fished by two-yeast hybrid into pGEX-4T-1 previously digested by *Eco*RI*.* pGEX-2TK -34, -14 and -4 kDa cath-D domains were obtained by inserting PCR-amplified cDNA encoding cath-D fragments into pGEX-4T-1 previously digested by *Eco*RI.

**Yeast two-hybrid assay.** Full-length 52 kDa precursor, 48 kDa intermediate, 34 kDa mature heavy chain, 14 kDa mature light chain and 4 kDa pro-fragment of human cath-D were each fused with the LexA DNA-binding domain in the pMW101 vector. A cDNA library derived from normal breast tissue was cloned into the galactosidase-inducible pYESTrp2 vector (Invitrogen) containing B42 activation domain. The yeast two-hybrid screen was performed as described previously (Slentz-Kesler et al., 2000).

**Transfections and outgrowth assays.** PEA13 cells were co-transfected with a 10-fold excess of wild-type cath-D or empty pSG5-neo vector and a pTK-Hyg hygromycin-resistance expression vector (Clontech) using Lipofectamine (Invitrogen). Colonies growing in the presence of hygromycin B (100 µg/ml, Invitrogen) were pooled and human cath-D was quantified using an immunoradiometric assay (Glondu et al., 2001). 30,000 HMF cells were plated in 6-well plates and after 3 days cells were transiently transfected with 10 µg human LRP1 or Luc siRNAs using Oligofectamine (Invitrogen). 35,000 MDA-MB-231 cells were plated in 12-well plates and after 2 days were transiently transfected with 2 µg human LRP1 or Luc siRNAs. In other experiments, 50,000 CD55-/-cath-D cells were plated in 12-well plates and after 2 days were transiently transfected with 2.5 µg mouse LRP1 or Luc siRNAs. For outgrowth assays, 50,000 CD55-/-SV40, CD55-/- cath-D, PEA10, PEA13 and PEA13-cath-D cells were re-suspended at 4°C in Matrigel (0.2 ml, 10 mg/ml) (BD Biosciences) and quickly added to a pre-set layer of Matrigel in 24-well plates (Glondu et al., 2001). Outgrowth assays were also performed with HMF, MDA-MB-231 and CD55-/-cath-D cells, 24 or 48 h post-transfection with LRP or Luc siRNAs. In co-culture outgrowth assays, 100,000 cells from mock- or cath-D-transfected 3Y1-Ad12 cell lines were plated in 24-well plates. After 2 days, cells were covered first with a layer of Matrigel, then with a layer of Matrigel containing 50,000 HMF fibroblasts 48 h post-transfection with LRP or Luc siRNAs (Laurent-Matha et al., 2005).
**siRNAs.** Duplexes of 21-nucleotide human LRP1 siRNA1 (target sequence AAGCAGTTTGCCTGCAGAGAT (SEQ ID NO: 6), residues 666-684) (Li et al., 2003), human LRP1 siRNA2 (target sequence AAGCTATGAGTTTAAGAAGTT (SEQ ID NO: 7)) (# D-040764-04-0010 Dharmacon), mouse LRP1 siRNA3 (target sequence AAGCAUCUCAGUAGACUAUCA (SEQ ID NO: 8)) (Fears and al., 2005), mouse LRP1 siRNA4 (target sequence AAGCAGTTTGCCTGCAGAGAC (SEQ ID NO: 9)) or firefly luciferase (Luc) siRNA (target sequence AACGTACGCGGAATACTTCGA (SEQ ID NO: 10)) were synthesized by MWG Biotech S.A. (France) or Dharmacon.

**GST pull-down assays.** [³⁵S]methionine-labelled pro-cath-D or LRP1β(1-476) were obtained by transcription and translation of pcDNA3.1(-)cath-D or pcONA3.1(+)LRP1β extracellular domain(1-476) expression plasmids, respectively, using the TNT^{T7}-coupled reticulocyte lysate system (Promega). GST, GST-LRP1β (307-479), GST-LRP1β shorter fragments, GST-cath-D-52K, GST-cath-D-34K, GST-cath-D-14K and GST-cath-D-pro-domain fusion proteins were produced in *Escherichia coli B* strain BL21 using isopropyl-1-thio-β-D-galactopyranoside (1 mM) for 3 h at 37°C. GST fusion proteins were purified on glutathione-Sepharose beads (Amersham Biosciences). For pull-down assays, 20 µl of bed volume of glutathione-Sepharose beads with immobilized GST fusion proteins were incubated overnight at 4°C with either [³⁵S]methionine-labelled pro-cath-D or LRP1β(1-476) in 500 µl PDB buffer (20 mM HEPES-KOH [pH 7.9], 10% glycerol, 100 mM KCI, 5 mM MgCl₂, 0.2 mM EDTA, 1 mM DTT, 0.2 mM phenylmethylsulfonyl fluoride) containing 15 mg/ml BSA and 0.1% Tween 20. Beads were washed four times with 500 µl PDB buffer, and bound proteins were resolved by 15% SDS-PAGE, stained with Coomassie blue, and exposed to autoradiographic film.

**Co-transfection and co-immunoprecipitation.** COS-1 cells were transfected at 100% confluency with 10 µg of pcDNA3-Myc- LRP1β or LRP1-IV-HA mini-receptor with or without 10 µg pcDNA3.1(-) or pcDNA3.1(-)cath-D vectors. Transient transfections were carried out using Lipofectamine and Opti-MEM (Gibco-BRL). Two days post-transfection, cells were lysed in 3 ml 50 mM Hepes [pH 7.5], 150 mM NaCl, 10% glycerol, 1% Triton X-100, 1.5 mM MgCl₂ 1 mM EGTA, 100 mM NaF, 10 mM sodium pyrophosphate, 500 µM sodium vanadate, 1 mM phenylmethylsulfonyl fluoride and a protease inhibitor cocktail (PLC lysis buffer). Lysates were incubated with 3 µg anti-cath-D M1 G8 monoclonal antibody or 40 µl anti-LRP1 11 H4 hybridoma overnight at 4°C, and subsequently with 25 µl 10% protein A- or G-Sepharose, respectively, for 2 h at 4°C on an agitator. Sepharose beads were washed 4 times with PLC buffer, boiled for 3 min in SDS sample buffer and resolved by SDS-PAGE.

**lmmunoprecipitation of endocytosed cath-D.** For cath-D endocytosis experiments, human cath-D transfected 3Y1-Ad12 cells (Glondu et al., 2001) were labelled with 175 µCi/ml [³⁵S]Translabel (MP Biomedicals, Inc.) for 24 h in serum, methionine and cysteine free DEM medium, and the labelled conditioned culture medium containing secreted pro-cath-D was used directly for internalization studies. 100,000 HMF cells were plated in 6-well plates and after 3 days cells were transiently transfected with 10 µg human LRP1 or Luc siRNAs using Oligofectamine (Invitrogen). Two days post-transfection, expression of human LRP1 (100 µg cellular protein) was monitored by Western blotting using the 11 H4 hybridoma. In parallel, HMF fibroblasts were incubated for 24 h in DEM medium supplemented with the conditioned medium prepared from 3Y1-Ad12-cath-D cells corresponding to 3 x 10⁶ cpm of total TCA-precipitable proteins, 10% FCS, 10 mM of methionine and 5 mM of cysteine. After incubation, the medium was discarded and cellular extracts were prepared. ³⁵S-Cath-D endocytosed into cells was immunoprecipitated with M1G8 anti-cath-D antibody and analyzed by 15% SDS-PAGE and autoradiography (Capony et al., 1987).

**Immunoblotting.** Cells grown to 80% confluency were lysed in PLC buffer. Equal amounts of protein (100 µg) from cell extracts quantitated by the Bradford assay, or equal volumes (80 µl) of conditioned media, were separated by SDS-PAGE on a 7.5 % gel for anti-LRP1β immunoblotting or on a 12 % gel for anti-cath-D immuno-blotting. Proteins were electro-transferred to PVDF membranes (VWR) and incubated with 11 H4 anti-LRP1β hybridoma (1/10 dilution), 1 µg/ml anti-human cath-D, 1 µg/ml anti-HA (12CA5 clone, Roche), 1 µg/ml anti-GAPDH (Santa Cruz Biotechnology, Inc.) monoclonal antibodies, or anti-ßactin polyclonal antibody (A2066, Sigma) (1/1000 dilution). Proteins were visualized with horseradish peroxidase-conjugated sheep anti-mouse immunoglobulin (Amersham) followed by the enhanced chemiluminescence system ECL (Perkin Life Sciences).

**Isolation of lipid rafts.** COS cells grown in 100 mm Petri dishes and transfected as described above were lysed in MEB buffer (150 mM NaCl, 20 mM Morpholine Ethane Sulfonic acid, [pH 6.5]) containing 1% Triton X100, 1 mM Na₃VO₄, 50 mM NaF, and protease inhibitor cocktail, and were homogenized with a dounce homogenizer. The preparation (4 ml) was mixed with an equal volume of 80% sucrose solution in MEB buffer in an ultracentrifuge tube over-layed with 4 ml of 30% sucrose and 1.5 ml of 5 % sucrose, and centrifuged at 39 000 rpm for 16 h at 4°C in a Beckman SW40 Ti. After centrifugation, 8 fractions (1 ml each) were collected from the top of each tube. An equivalent aliquot of each fraction was subjected to immunoblot analysis.

**lmmunocytochemistry.** COS cells grown in 12-well plates and transfected as previously described were fixed with 4 % paraformaldehyde, permeabilized with 0.1% Triton X100, and blocked with 2.5 % goat serum (Sigma). Cells were then incubated with an anti-LRP1β rabbit polyclonal antibody (1/200) followed by a FITC-conjugated goat anti-rabbit IgG (1/200; Jackon ImmunoResearch). After washing, cells were incubated with an anti-cath-D M2E8 mouse monoclonal (40 µg/ml) followed by an AlexaFluor 568-conjugated goat anti-mouse IgG (1/200; Invitrogen) and were observed by confocal microscopy using a BioRad 1024 CLSM system with a 60X (1.4NA) Nikon objectif.

**Immunogold.** Cells were fixed with 3% paraformaldehyde and 0.1% glutaraldehyde in a phosphate buffer [pH 7.2] overnight at 4°C and were then cryoprotected. Ultra-thin sections (85 nm) were incubated in PBS first with 0.1 % cold water fish gelatin, 1 % BSA, and 0.05 % Tween 20 (incubation buffer), then with rabbit anti-LRP1β (1/20) and mouse anti-cath-D M2E8 (4 µg/ml), and subsequently with 15 nm goat anti-rabbit-gold and 6 nm goat anti-mouse-gold 5 (Aurion) diluted 1/20. Sections were observed using a Hitachi 7100 transmission electron microscope.

**LRP1 ectodomain secretion assay.** COS cells plated in 12-well plates were transfected with a β-galactosidase reporter, LRP1β-fused N-terminally to secreted alkaline phosphatase (SEAP) and either empty or cath-D vectors. The medium was changed 24 h later, and then collected after another 24 h. Measurement of SEAP activity in the conditioned media was carried out in duplicate by chemiluminescent assay (Roche Applied Science). SEAP activity was normalized to µ-galactosidase activity measured by hydrolysis of *o*-nitrophenyl-β-D-galactopyranoside in cells lysed in the reporter lysis buffer (Promega).

**RNA extraction, RT-PCR and Q-PCR.** RNA extraction and reverse transcription were performed and Q-PCR was carried out using a LightCycler and the DNA double strand specific SYBR Green I dye for detection (Roche). Q-PCR was performed using gene-specific oligonucleotides and results were normalized to RS9 levels.

### RESULTS

**A yeast two-hybrid approach identified cath-D as a ligand for the extracellular domain of the LRP1** β **chain.** To identify additional cath-D receptors, we have performed yeast two-hybrid screens using LexA DNA-binding domain fusion proteins containing various fragments of cath-D as bait and a cDNA library isolated from normal breast tissue fused to the transactivation domain of B42. Results from the initial yeast two-hybrid screen were confirmed by one-on-one two-hybrid assays and indicates that human cath-D binds to the β chain of LRP1. The clone we isolated in our screen is 100% identical to amino acids 307-479 of the β chain of LRP1 (Fig. 1A). The interaction was observed with the 48 kDa intermediate form as well as with both the 34 kDa and the 14 kDa polypeptides of cath-D indicating that the interaction interface spans both sub-units (Fig. 1 B). No interaction could be detected with either the 52 kDa precursor, or the 4 kDa pro-fragment.

**Cath-D associates *in vitro* with the extracellular domain of the** β **chain of LRP1.** We have confirmed the direct interaction between cath-D and the LRP1 β chain using GST pull-down assays. GST-fusion proteins containing amino acids 307-479 of the extracellular domain of the LRP1 β chain (Fig. 1C panel a, GST-LRP1β (307-479)) were immobilized on glutathione-agarose beads and incubated with full-length pro-cath-D, labelled with ³⁵S-Met by *in vitro* translation. The results show binding of pro-cath-D to GST-LRP1β (307-479) (Fig. 1C, panel b). Subsequently, the 52, 34, 14 and 4 kDa cath-D fragments were expressed as GST fusion proteins (Fig. 1D, panels a and c) and tested for their ability to bind to the full-length extracellular domain of LRP1β (1-476). LRP1β (1-476) was shown to bind specifically to both the 52, 34 and 14 kDa cath-D polypeptides (Fig. 1D, panels b and d). In contrast, GST-RAP (Fig. 1E, panel a) was shown to bind poorly to LRP1β (1-476) (Fig.1E, panel b) and did not compete for the binding with pro-cath-D (Fig. 1 F). In order to define the binding site, GST-fusion proteins containing fragments of LRP1 β (307-479) were tested for their ability to bind to pro-cath-D (Figs. 2A and 2B). We identified fragment 4 (F4, 349-394) as the shortest fragment that bound to cath-D (Fig. 2B, panel b). Finally, a peptide composed of residues 349-397, spanning LRP1 EGF-like repeat 20 (Fig. 2C, panel a) competed efficiently for the binding of LRP1β (1-476) to GST-pro-cath-D (Fig. 2C, panel b). These results provide independent confirmation of the yeast two-hybrid results and demonstrate a direct interaction between cath-D and residues 349-394 in the extracellular domain of the LRP1 β chain. The chaperone protein RAP does not compete for binding of cath-D to the LRP1.

**Pro-cath-D and the** β **chain of LRP1 associate *in vivo* and co-distribute in cells**. To determine whether pro-cath-D associates with LRP1 β chain *in vivo,* we used a cDNA encoding Myc-LRP1β in which the β chain of LRP1 was linked at the amino-terminus to a signal peptide and a Myc epitope (Barnes et al., 2003). Myc-LRP1β was expressed in COS cells in absence or presence of wild-type or D231 N mutated cath-D. Our results show that both wild-type and D231 N cath-D precursor co-immunoprecipitate with the β chain of LRP1 (Fig. 3A, panel a). ^{D231N}cath-D displayed an increased electrophoretic mobility, corresponding to a shift of about 1 kDa in the apparent molecular mass (Fig. 3A, panel a), as previously described (Laurent-Matha et al., 2006). A longer gel exposure revealed that the 48 and 34 kDa cath-D polypeptides also co-immunoprecipitate with LRP1β (Fig. 3A, panel c). These results suggest that the LRP1 β chain interacts preferentially with the 52 kDa cath-D precursor (Fig. 3A, panel a). Moreover, pro-cath-D and the associated LRP1 β chain can be directed to the lysosomes, as revealed by co-immunoprecipitation of LRP1 β chain with the 48 and 34 kDa forms of cath-D (Fig. 3A, panel c). Reciprocally, anti-LRP1 β chain immunoblotting of anti-cath-D immunoprecipitate confirmed that the β chain of LRP1 co-immunoprecipitates with cath-D and ^{D231N}cath-D (Fig. 3A, panel b). Together, these results demonstrate that the pro-cath-D precursor, as well its mature forms, interact with the β chain of LRP1 in cells. Finally, both pro-cath-D (Fig. 3A, panel d) and Myc-LRP1β (Fig. 3A, panel e) co-immunoprecipitated with endogenous LRP1α chain, suggesting that pro-cath-D bound to LRP1β interacts with LRP1α. We therefore postulate that cath-D, LRP1β and LRP1 α can associate in cells.
To confirm the ability of pro-cath-D to bind to the β chain of LRP1 in the presence of the α chain, we then performed co-immunoprecipitation studies using a LRP1-IV-HA mini-receptor containing the β chain and 120 kDa of the α chain, corresponding to the entire cluster IV of class A ligand binding repeats and tagged with an HA epitope (Zhang et al., 2004). Anti-cath-D immunoblotting of anti-LRP1 β chain immunoprecipitates confirmed that both wild-type and D231 N pro-cath-D co-immunoprecipitated the β chain of the LRP1-IV-HA mini-receptor (Fig. 3B, panel a). The β chain of the LRP1-IV-HA mini-receptor appears to co-immunoprecipitate with pro-cath-D less efficiently as compared to Myc-LRP1β (Fig. 3A, panel a). This difference may also come from different transfection efficiency since the level of expression of transfected LRP1-IV-HA was 7 times lower as compared to that of transfected Myc-LRP1β (Fig. 3B, panel d). Moreover, anti-cath-D immunoblotting of anti-HA immunoprecipitates (Fig. 3B, panel b) as well as anti-HA immunoblotting of anti-cath-D immunoprecipitates (Fig. 3B, panel c) revealed that the α chain of LRP1-IV-HA mini-receptor co-immunoprecipitates with pro-cath-D. These results indicate that cath-D, LRP1β and LRP1α-IV associate in cells. Unexpectedly, anti-HA immunoblotting of anti-LRP1 β immunoprecipitate failed to co-immunoprecipitate LRP1 β (Fig. 3B, panel c). Altogether, our experiments strongly suggest that pro-cath-D can interact with LRP1β in the context of a complex between pro-cath-D, LRP1β and LRP1 α.

**Pro-cath-D and LRP1 co-localize partially in cells.** To study co-localization, COS cells transiently co-expressing cath-D and Myc-LRP1β were analyzed by confocal microscopy after double staining with a monoclonal antibody specific for the pro-cath-D precursor and a polyclonal antibody specific for the cytoplasmic tail of the LRP1 β. Figure 4A shows that a fraction of pro-cath-D (in red) was co-localized with LRP1β (in green) in punctuate structures. Pro-cath-D also partially co-localized with LRP1β in the presence of 10 mM M6P, known to prevent its endocytosis via the M6P receptors (Fig. 4A, panels e-i), suggesting that pro-cath-D/LRP1β partial co-localization was not due to vesicular co-transport of these two proteins (e.g. co-endocytosis of pro-cath-D via M6P receptors and endocytosis of LRP1). Cellular co-localization of LRP1 and pro-cath-D was further confirmed by immunogold electron microscopy (Fig. 4B). Co-localization of pro-cath-D and β chain of LRP1 was observed at the cell surface (Fig. 4B, panel a-c), as well as in vesicular-like structures (Fig. 4B, panel d). Co-localization was also detected with COS cells transiently transfected with Myc-LRP1β vector and incubated with 15 nM pro-cath-D in the presence of 10 mM M6P (data not shown). Together with the immunofluorescence studies, these results indicate that pro-cath-D and the β chain of LRP1 partially co-localize in intact cells.
At the cell surface, LRP1 has been described to partition into both clathrin-coated pits and lipid rafts. Depending on the cell types, it is principally located to clathrin coated pits (Zhang et al., 2004) or in caveolae membranes like in fibroblasts (Boucher et al., 2002). A recent study describes that LRP1 only transiently associates with lipid rafts (Wu and Gonias, 2005). We next investigated whether pro-cath-D might be present together with the LRP1β chain in lipid rafts. COS cells expressing cath-D either in the absence (Fig. 4C, panel a) or the presence of Myc-LRP1β (Fig. 4C, panel b) were subjected to sucrose density ultracentrifugation. The lipid marker ganglioside GM1 of rafts was found predominantly in low density fractions 3-6 (Fig. 4C, panels a and b, bottom panels). Endogenous LRP1β was detected in both raft-rich and non-raft fractions (Fig. 4C, panel a, top panel). Pro-cath-D was mainly located in the non-raft fractions 7 and 8, even through some pro-cath-D and its 48 kDa intermediate form were also detected in the raft-rich fractions 5 and 6 (Fig. 4C, panel a, medium panel). Overexpression of Myc-LRP1β, which localized largely to the raft-rich fractions (Fig. 4C, panel b, top panel), resulted in a large increase in the presence of pro-cath-D and its neo-processed forms (e.g. early intermediates previously described (Laurent-Matha et al., 2006) as well as the 48 kDa intermediate) in those same raft-rich fractions (Fig 4C panel b, medium panel). Together our results show a partial co-localization of pro-cath-D and LRP1 β chain at the cell surface, especially in lipid rafts, as well as in vesicular-like structures.

**Cath-D triggers LRP1 RIP.** It has been recently shown that the β chain of LRP1 can undergo ectodomain shedding by the β-secretase type 1 membrane-associated aspartic protease BACE1, followed by intracellular cleavage by γ-secretases, in a process called RIP (Regulated Intramembrane Proteolysis) (von Arnim et al., 2005). To investigate whether cath-D may induce LRP1β ectodomain shedding, we used a cDNA encoding SEAP-LRP1β in which the β chain of LRP1 was fused to the carboxy-terminus of secreted alkaline phosphatase (SEAP) (von Arnim et al., 2005). The fusion protein was transiently expressed in COS cells in the presence or absence of wild-type or D231 N cath-D and SEAP activity was quantified in the culture supernatant as a measure of LRP1β ectodomain shedding (von Arnim et al., 2005). The presence of cath-D led to a 2.5 fold increase in SEAT-LRP1 ectodomain shedding when compare to controls (Fig. 5A, panel a). Pretreatment of cath-D expressing cells with the aspartic protease inhibitor, pepstatin A, prevented SEAP-LRP1β ectodomain shedding (Fig. 5A, panel b). Cleavage specificity was also confirmed by co-transfecting cath-D with an anti-cath-D siRNA (Bidère et al., 2003) that led to an inhibition of SEAP-LRP1β cleavage (Fig. 5A, panel b). Interestingly, treatment with anti-cath-D siRNA generated inhibition to levels below those seen in control suggesting that endogenous cath-D also triggers SEAP-LRP1 β chain shedding. Catalytically-inactive ^{D231N}cath-D was inefficient in cleaving SEAP-LRP1β and seemed to behave as a dominant negative since SEAP activity was 3-fold reduced in its presence relative to the control level (Fig. 5A, panel b). To explore LRP1 β ectodomain shedding by cath-D, we finally studied whether cath-D can cleave within its LRP1β extracellular interaction domain (see Fig. 2). Three cath-D cleavage sites were identified within the EGF-like repeat 20 (349-397) of LRP1 β (Fig. 5B), suggesting that cath-D may generate LRP1 β ectodomain shedding by cleaving within its interaction domain.
To further investigate the cath-D-induced LRP1 cleavage, Myc-LRP1β was expressed in COS cell in the absence or presence of wild-type or ^{D231N}cath-D. Analysis of anti-LRP1 immunoprecipitates by anti-LRP1 immunoblotting revealed the presence of a 25 kDa protein that most likely represents the membrane-associated product of LRP1β ectodomain shedding (LRP1β-CTF, Fig. 5C). Co-expression with wild-type cath-D resulted in an increase in the levels of LRP1β-CTF whereas ^{D231N}cath-D had no effect (Fig. 5C). In the presence of DAPT, an inhibitor of γ-secretases, a strong increase in the presence of LRP1β-CTF (Fig. 5C) was observed indicating its further cleavage by a γ-secretases. We were unable to detect soluble LRP1β intracellular fragment (LRP1 β-ICD), suggesting that this fragment was rapidly degraded following its release from the membrane as previously described with LRP1B (Liu et al., 2007). Our results are consistent with a cath-D-mediated LRP1 β chain ectodomain shedding resulting in the generation of a 25 kDa carboxyl-terminal membrane associated LRP1 β chain fragment (LRP1β-CTF). This fragment is subsequently cleaved by a γ-secretase-like activity causing the release of the soluble LRP1-ICD cytoplasmic fragment. We therefore propose that cath-D can initiate LRP1 RIP.

**LRP1 is highly expressed in fibroblasts.** To examine the functional significance of the interaction between cath-D and the LRP1 β chain , we first screened a variety of cell lines for LRP1 and cath-D expressions (Fig. Sup1). Our survey suggests that LRP1 is highly expressed in fibroblast cell lines and this conclusion is consistent with the data obtained from cancer biopsies (Christensen et al., 1996; Obermeyer et al., 2007). In contrast, breast cancer cells express low levels of the LRP1 β chain while over-secreting pro-cath-D (Capony et al., 1989).

**Cath-D interacts with endogenous LRP1 in fibroblasts.** To determine whether cath-D interacts with LRP1 endogenously expressed in fibroblasts, we then asked whether internalization of pro-cath D was dependent on the presence of LRP1. Endocytosis of secreted labelled pro-cath-D was analyzed in HMF fibroblasts in which endogenous LRP1 expression was inhibited by siRNA1 (Fig. 6A; see Fig. Sup. 3A, panel a). As previously described (Capony et al., 1987), labelled 52 kDa pro-enzyme was transformed into a 48 kDa intermediate and 34 kDa mature enzyme following binding and internalization (Fig. 6A, panel a, *lane 1*). As expected, the presence of excess soluble M6P prevented cath-D binding to M6P receptors (Capony et al., 1987) and inhibited pro-cath-D internalization over 3 h by 82 % (Fig. 6A, panel a, *compare lanes 1 and 3*). Thus, 18 % of pro-cath D was taken up in a manner that is independent of M6P receptors (Fig. 6A, panel a, *lane 3*), as previously described for mouse fibroblasts (Laurent-Matha et al., 2005). Interestingly, inhibition of LRP1 expression (Fig. 6A, panel b) led to a 50 % decrease of pro-cath-D uptake in the presence of M6P (Fig. 6A, panel *a, compare lanes 3 and 4*). We also observed a significant decrease in pro-cath-D internalization in the absence of LRP1 when internalization was measured over an 18 h period (Fig. 6A, panel c, *compare lanes 3 and 4,* and panel d). These findings together with those obtained from co-localization experiments (Fig. 4) suggest that pro-cath-D is endocytosed in part *via* LRP1. We finally investigated whether pro-cath-D that is being internalized by HMF fibroblasts can induce LRP1 RIP (Fig. 6B, panel a). Pro-cath-D (12 nM) added to HMF fibroblasts for 24 hours caused an increase in the levels of LRP1β-CTF. Addition of the γ-secretase inhibitor DAPT led to a further increase in the level of membrane bound LRP1β-CTF, that was significantly higher in the presence of pro-cath-D as compared to control (161.8 % +/- 6.6 (n = 4), p< 0.005, Student'*t* test). In parallel, we also observed the presence of a 68 kDa LRP1β form in the culture media of HMF cells incubated for 24 h with 12 nM pro-cath-D that may correspond to shedded LRP1β ectodomain (Fig. 6B, panel b). Together, our results indicate that, in human mammary fibroblasts, pro-cath-D interacting with endogenous LRP1 is partially endocytosed via this receptor, and suggest, for the first time, that cath-D may initiate LRP1 RIP in the breast tumor micro-environment context.

**LRP1 is a mediator of cath-D stimulation of fibroblast invasive growth.** We first studied whether LRP1 expression was necessary for basal outgrowth of HMF human mammary fibroblasts in 3D Matrigel. As previously published (Laurent-Matha et al., 2005), HMF transfected with control Luc siRNA grew poorly in Matrigel and adopted an elongated morphology typical for fibroblasts (Fig. Sup. 2, panels a, f). Inhibition of LRP1 expression by siRNA1 and siRNA2 (Fig. Sup. 2, panels c and h; Fig. Sup. 3A, panels a and b) led to a rounded morphology and to a decrease of the protrusions (Fig. Sup. 2, panels b (*see inset*) and g). Cell viability of HMF cell embedded in Matrigel was not altered by LRP1-silencing. This was shown by p-nitrotetrazolium staining of cell in Matrigel (Fig. Sup. 2, panels d and e) and by cell cycle analyses of cells extracted from Matrigel by flow cytometry (S+G2M = 17 % for Luc siRNA HMF cells; S+G2M = 19.7 % for LRP1 siRNA1 HMF cells). These data suggest that LRP1 is required for typical fibroblastic morphology in three-dimensional matrices.
To gain further insight as to whether LRP1 mediates the paracrine action of secreted pro-cath-D, we next performed Matrigel assays with cath-D-transfected and LRP1-silenced fibroblasts. As previously published (Laurent-Matha et al., 2005), (CD55-/-cath-D) cath-D-expressing fibroblasts proliferated efficiently in Matrigel, whereas (CD55-/- SV40) cath-D-deficient fibroblasts grew poorly (Fig. 7A, panels a and b). Inhibition of LRP1 expression in CD55-/-cath-D fibroblasts by siRNA3 and siRNA4 (Fig. 7A, panels e, h; Fig. Sup. 2B, panels a and b) strongly inhibited their cath-D-dependent outgrowth (Fig. 7A, panels c, d, f and g). These results indicate that cath-D requires LRP1 to promote fibroblast invasive growth. To further support this idea, we performed 3D co-culture assays of control and LRP1-deficient HMF fibroblasts (Fig. 7B, panel f) and control or cath-D-expressing 3Y1-Ad12 cancer cell lines (Fig. 7B, panel e). Only pro-cath-D secreting 3Y1-Ad12 cells stimulated the outgrowth of HMF fibroblasts (Fig. 7B, panel b), whereas 3Y1-Ad12 control cells had no effect (Fig. 7B, panel a). Inhibiting LRP1 expression in HMF fibroblasts using siRNA1 (Fig. 7B, panel f) blocked their response to secreted pro-cath-D (Fig. 7B, panel d). Similar results were obtained with LRP1 siRNA2 (Fig. Sup.4). These results support a model in which pro-cath-D secreted by epithelial cancer cells stimulates, in a paracrine and LRP1-dependent manner, the outgrowth of surrounding fibroblasts.

### DISCUSSION

In this example, we have described a physical interaction between cath-D and the extracellular domain of the LRP1 β chain that occurs at the cell surface in lipid rafts as well as in vesicular-like structures that resemble the endosomal compartments. We propose a model (Fig. 8) in which pro-cath-D binds to residues 349-394 in the extracellular region of the β chain of LRP1, possibly in lipid rafts, new portals for endocytosis (Parton and Richards, 2003). Upon binding, pro-cath-D is internalized and, due to the acidification within the early endosomes, is processed. Processing leads to the activation of cath-D. Cath-D then cleaves LRP1 at 3 major sites within the 349-394 binding domain in the β chain. Shed LRP1 ectodomain is then released in the serum through recycling vesicles. Ectodomain shedding results in the production of a 25 kDa membrane-bound fragment (LRP1β-CTF), that is further cleaved by γ-secretases, leading to the production of LRP1β-ICD, that might affect gene transcription according to previous reports (May et al., 2002; von Arnim et al., 2005). Our results therefore support a model in which pro-cath-D that is secreted by breast cancer cells acts as an extracellular messenger that signals by binding to LRP1 present on the surface of fibroblasts.
Cath-D is an aspartic protease that is overexpressed and secreted in the extracellular environment by human epithelial breast cancer cells (Westley and Rochefort, 1980; Vignon et al., 1986; Capony et al., 1987; Capony et al., 1989). Cath-D stimulates tumor cell proliferation, fibroblast invasive growth, and tumor angiogenesis (Vignon et al., 1986; Garcia et al., 1990; Vetvicka et al., 1994; Liaudet et al., 1994; Liaudet et al., 1995; Glondu et al., 2001; Glondu et al., 2002; Berchem et al., 2002; Laurent-Matha et al., 2005). It is well established that M6P receptors bind and can mediate pro-cath-D uptake into cells (Vignon et al., 1986; Von Figura and Hasilik, 1986; Laurent-Matha et al., 1998; Heylen et al., 2002). However, the realization that M6P receptors do not mediate the cath-D mitogenic effects has led to a search for alternative cath-D receptors (Fusek and Vetvicka, 1994; Glondu et al., 2001; Rochefort and Liaudet-Coopman, 1999; Benes et al., 2006; Liaudet-Coopman et al., 2006). In particular, the existence of a receptor binding to the cath-D pro-domain has been proposed (Fusek and Vetvicka, 1994; Vetvicka et al., 1999; Benes et al., 2006). Our present work describes the discovery of LRP1 as a new receptor and substrate for cath-D. Previous work, using the RAP chaperon protein (Herz et al., 2006) that competes with ligands for binding to α chain of LRP1 (Laurent-Matha et al., 2002), excluded LRP1 as a cath-D receptor. Our observations showing that cath-D binds to the LRP1 β chain explain why RAP does not compete with cath-D for binding to LRP1.
LRP1 was originally identified as an internalization receptor that is related to the LDL receptor (Herz and Strickland, 2001). Recently, it has become clear that LRP1 may be involved in signal transduction by protein tyrosine kinases (Herz and Strickland, 2001). Tyrosine phosphorylation of the NPXY motifs that are present in the cytoplasmic region of LRP1 has been observed in response to PDGF-BB (Loukinova et al., 2002; Boucher et al., 2002; Boucher et al., 2003; Boucher and Gotthardt, 2004; Newton et al., 2005), CTGF (Yang et al., 2004), or tPA (Hu et al., 2006) and in fibroblasts transformed with v-Src (Barnes et al., 2001; Barnes et al., 2003). While we did not observe any tyrosine phosphorylation of LRP1 in response to cath-D, we did observe that the interaction with cath-D resulted in proteolytic processing of LRP1, a process that is now generally referred to as RIP.
RIP is a process that involves two cleavage events (Brown et al., 2000). The first cleavage event occurs in the extracellular region close the plasma membrane and results in the release of the extracellular region, a process also known as ectodomain shedding. Ectodomain shedding leaves behind an integral membrane protein with a very short extracellular domain that is subject to proteolytical cleavage within its transmembrane domain. Intramembrane cleavage can be carried out by a small group of enzymes that include γ-secretases (Kopan and Ilagan, 2004). Upon release into the cytosol, cytoplasmic fragments can interact with intracellular signalling proteins, diffuse to nucleus and control gene transcription (Brown et al., 2000).

The results, showing that binding of cath-D induces LRP1 RIP, are consistent with the following observations. A soluble fragment of LRP1, composed of the α chain and the extracellular part of the β chain, has been detected in the circulation (Quinn et al., 1997; Quinn et al., 1999). LRP1β ectodomain can be cleaved by the β-secretase type I membrane-associated aspartic protease BACE1 (Von Arnim et al., 2005), previously implicated in APP RIP and Alzheimer disease (Vassar et al., 1999). LRP1 can then act as a substrate for γ-secretases (May et al., 2002; Von Arnim et al., 2005). When expressed by itself, the cytoplasmic domain of LRP1 localizes to the nucleus where it can associate with Fe65 and Tip60 (Konishita et al., 2003). Together these observations support the notion that LRP1 is subject to RIP and our results strongly suggest that LRP1 RIP may be induced, not only by BACE1 in the neuronal pathology (Von Arnim et al., 2005), but as well by cath-D in the breast tumor micro-environment.
LRP1 has been localized not only to clathrin coated pits, but also to lipid rafts either transiently in the absence of a stimulus (Wu et al., 2005) or in response to PDGF-BB or insulin (Boucher et al., 2002; Zhang et al., 2004). It is now well recognized that BACE 1 and γ-secretases are also highly enriched in lipid raft micro-domains (Vetrivel et al., 2004), suggesting that LRP1 RIP may take place in this particular micro-environment. In the present study, we observed that endogenous LRP1β localized partially in lipid rafts and that its enrichment by transfection led to a concomitant increase of LRP1β and pro-cath-D and cath-D neo-processed forms in these lipid micro-domains. It is worthy to note that this study revealed, for the first time, the presence of pro-cath-D in lipid rafts even in the absence of transfected LRP1β. The lysosomal cysteine protease cath-B also localizes within these lipid micro-domains in association with the annexin II heterotetramer (Mohamed and Sloane, 2006).
While both wild-type and D231 N mutant pro-cath-D were shown to bind to the LRP1 β chain, we observed stronger binding with catalytically inactive mutant. The fact that mutated pro-cath-D also bound to LRP1 is consistent with previous studies (Glondu et al., 2001; Berchem et al., 2002; Laurent-Matha et al., 2005). The fact that proteolytically-inactive ^{D231N}cath-D did not cause LRP1 ectodomain shedding explains why we observe increased co-immunoprecipitation with this mutant. It is not fully understood whether LRP1 cleavage is required for cath-D biological activity. The observation that catalytically inactive cath-D induces invasive outgrowth of fibroblasts suggests that it is not. We therefore propose that cath-D binding to LRP1 may lead to the activation of one or more intracellular signalling pathways that elicit fibroblast invasive growth.
Previous reports have described variable effects of LRP1 on proliferation, motility or invasion (Lillis et al., 2005). Discrepancies in the results may be based on differences in the ligands or cell lines that were used in those studies (Lillis et al., 2005). Here, we describe a role for LRP1 as a positive regulator of the invasive outgrowth of mammary tissue fibroblasts that is seen in response to pro-cath-D. Indeed, suppression of LRP1 expression using RNA interference resulted in an abrogation of cath-D-dependent invasive growth. Because LRP1 is expressed on fibroblasts in breast cancer biopsies and on the invasive front of fibroblasts in colon cancer biopsies (Christensen et al., 1996, Obermeyer et al., 2007), and because the C766T LRP1 gene polymorphism is associated with an increased risk of developing breast cancer (Benes et al., 2003), LRP1 may be involved in the onset and progression of various human malignancies
Others and we believe that cath-D is involved in creating the micro-environment that can sustain breast tumor growth and progression. As such, cath-D, in addition to be a prognostic marker for breast cancer, may become a potential target for therapeutic intervention. As such, the identification of a LRP1 peptide (349-397) that can inhibit pro-cath-D binding opens new therapeutic strategies. Our study further indicates that soluble LRP1 β chain ecto-domain may be detected in the circulation of breast cancer patients that overexpress cath-D. This may be used as a diagnostic tool for early detection of breast cancer.

### REFERENCES

All the references cited are incorporated herein by reference.
Annicotte JS, Fayard E, Swift GH, Selander L, Edlund H, Tanaka T, Kodama T, Schoonjans K, Auwerx J (2003) Pancreatic-duodenal homeobox 1 regulates expression of liver receptor homolog 1 during pancreas development. Mol Cell Biol 23: 6713-6724.
Barnes H, Ackermann EJ, van der Geer P (2003). v-Src induces Shc binding to tyrosine 63 in the cytoplasmic domain of the LDL receptor-related protein 1. Oncogene 22: 3589-3597.
Barnes H, Larsen B, Tyers M, and van Der Geer P (2001). Tyrosine-phosphorylated low density lipoprotein receptor-related protein 1 (Lrp1) associates with the adaptor protein SHC in SRC-transformed cells. J Biol Chem 276: 19119-19125.
Benes P, Jurajda M, Zaloudik J, Izakovicova-Holla L, Vacha J (2003). C766T low-density lipoprotein receptor-related protein 1 (LRP1) gene polymorphism and susceptibility to breast cancer. Breast Cancer Res 5, R77-81.
Benes P, Vashishta A, Saraswat-Ohri S, Fusek M, Pospisilova S, Tichy B, Vetvicka V (2006) Effect of procathepsin D activation peptide on gene expression of breast cancer cells. Cancer Lett 239 : 46-54.
Berchem G, Glondu M, Gleizes M, Brouillet JP, Vignon F, Garcia M, Liaudet-Coopman E (2002) Cathepsin-D affects multiple tumor progression steps in vivo: proliferation, angiogenesis and apoptosis. Oncogene 21, 5951-5955.
Bidere N, Lorenzo HK, Carmona S, Laforge M, Harper F, Dumont C, Senik A (2003) Cathepsin D triggers Bax activation, resulting in selective apoptosis-inducing factor (AIF) relocation in T lymphocytes entering the early commitment phase to apoptosis. J Biol Chem 278: 31401-31411.
Bossard N, Descotes F, Bremond AG, Bobin Y, De Saint Hilaire P, Golfier F, Awada A, Mathevet PM, Berrerd L, Barbier Y, Esteve J (2003) Keeping data continuous when analyzing the prognostic impact of a tumor marker: an example with cathepsin D in breast cancer. Breast Cancer Res Treat 82: 47-59.
Boucher P, Gotthardt M (2004) LRP and PDGF signaling: a pathway to atherosclerosis. Trends Cardiovasc Med 14: 55-60.
Boucher P, Gotthardt M, Li WP, Anderson RG, Herz J (2003) LRP: role in vascular wall integrity and protection from atherosclerosis. Science 300: 329-332.
Boucher P, Liu P, Gotthardt M, Hiesberger T, Anderson RG, Herz J (2002) Platelet-derived growth factor mediates tyrosine phosphorylation of the cytoplasmic domain of the low Density lipoprotein receptor-related protein in caveolae. J Biol Chem 277: 15507-15513.
Brown MS, Ye J, Rawson RB, Goldstein JL (2000) Regulated intramembrane proteolysis: a control mechanism conserved from bacteria to humans. Cell 100: 391-398.
Capony F, Rougeot C, Montcourrier P, Cavailles V, Salazar G, Rochefort H (1989) Increased secretion, altered processing, and glycosylation of pro-cathepsin D in human mammary cancer cells. Cancer Res 49: 3904-3909.
Capony F, Morisset M, Barrett AJ, Capony JP, Broquet P, Vignon F, Chambon M, Louisot P, Rochefort H (1987) Phosphorylation, glycosylation, and proteolytic activity of the 52-kD estrogen-induced protein secreted by MCF7 cells. J Cell Biol 104: 253-262.
Christensen L, Wiborg Simonsen AC, Heegaard C W, Moestrup SK, Andersen JA, Andreasen PA (1996) Immunohistochemical localization of urokinase-type plasminogen activator, type-1 plasminogen-activator inhibitor, urokinase receptor and alpha(2)-macroglobulin receptor in human breast carcinomas. Int J Cancer 66: 441-452.
De Strooper B, Annaert W, Cupers P, Saftig P, Craessaerts K, Mumm JS, Schroeter EH, Schrijvers V, Wolfe MS, Ray WJ, Goate A, Kopan R (1999) A presenilin-1-dependent gamma-secretase-like protease mediates release of Notch intracellular domain. Nature 398: 518-522.
De Strooper B, Saftig P, Craessaerts K, Vanderstichele H, Guhde G, Annaert W, Von Figura K, Van Leuven F (1998) Deficiency of presenilin-1 inhibits the normal cleavage of amyloid precursor protein. Nature 391: 387-390.
Dello Sbarba P, Rovida E (2002) Transmodulation of cell surface regulatory molecules via ectodomain shedding. Biol Chem 383: 69-83.
Faust PL, Kornfeld S, Chirgwin JM (1985) Cloning and sequence analysis of cDNA for human cathepsin D. Proc Natl Acad Sci U S A 82: 4910-4914.
Fears CY, Grammer JR, Stewart JE, Jr Annis DS, Mosher DF, Bornstein P, Gladson CL (2005) Low-density lipoprotein receptor-related protein contributes to the antiangiogenic activity of thrombospondin-2 in a murine glioma model. Cancer Res 65: 9338-9346.
Ferrandina G, Scambia G, Bardelli F, Benedetti Panici P, Mancuso S, Messori A (1997) Relationship between cathepsin-D content and disease-free survival in node-negative breast cancer patients: a meta-analysis. Br J Cancer 76: 661-666.
Foekens JA, Look MP, Bolt-de Vries J, Meijer-van Gelder ME, van Putten WL, Klijn JG (1999) Cathepsin-D in primary breast cancer: prognostic evaluation involving 2810 patients. Br J Cancer 79: 300-307.
Freiss G, Vignon F, Rochefort H (1988) Characterization and properties of two monoclonal antibodies specific for the Mr 52,000 precursor of cathepsin D in human breast cancer cells. Cancer Res 48: 3709-3715.
Fusek M, Vetvicka V (1994) Mitogenic function of human procathepsin D: the role of the propeptide. Biochem J 303: 775-780.
Garcia M, Capony F, Derocq D, Simon D, Pau B, Rochefort H (1985) Characterization of monoclonal antibodies to the estrogen-regulated Mr 52,000 glycoprotein and their use in MCF7 cells. Cancer Res 45: 709-716.
Garcia M, Derocq D, Pujol P, Rochefort H (1990) Overexpression of transfected cathepsin D in transformed cells increases their malignant phenotype and metastatic potency. Oncogene 5: 1809-1814.
Glondu M, Coopman P, Laurent-Matha V, Garcia M, Rochefort H, Liaudet-Coopman, E (2001) A mutated cathepsin-D devoid of its catalytic activity stimulates the growth of cancer cells. Oncogene 20: 6920-6929.
Glondu M, Liaudet-Coopman E, Derocq D, Platet N, Rochefort H, Garcia M (2002) Down-regulation of cathepsin-D expression by antisense gene transfer inhibits tumor growth and experimental lung metastasis of human breast cancer cells. Oncogene 21: 5127-5134.
Hass MR, Yankner BA (2005) A {gamma}-secretase-independent mechanism of signal transduction by the amyloid precursor protein. J Biol Chem 280: 36895-36904.
Hebert SS, Serneels L, Tolia A, Craessaerts K, Derks C, Filippov MA, Muller U, De Strooper B (2006) Regulated intramembrane proteolysis of amyloid precursor protein and regulation of expression of putative target genes. EMBO Rep 7: 739-745.
Herz J (2006) The switch on the RAPper's necklace. Mol Cell 23: 451-455.
Heylen, N., L.M. Vincent, V. Devos, V. Dubois, C. Remacle, and A. Trouet. 2002. Fibroblasts capture cathepsin D secreted by breast cancer cells: possible role in the regulation of the invasive process. Int. J. Cancer 20:761-767.
Hu K, Yang J, Tanaka S, Gonias SL, Mars WM, Liu Y (2006) Tissue-type plasminogen activator acts as a cytokine that triggers intracellular signal transduction and induces matrix metalloproteinase-9 gene expression. J Biol Chem 281: 2120-2127.
Kinoshita A, Shah T, Tangredi MM, Strickland DK, Hyman BT (2003) The intracellular domain of the low density lipoprotein receptor-related protein modulates transactivation mediated by amyloid precursor protein and Fe65. J Biol Chem 278: 41182-41188.
Laurent-Matha V, Derocq D, Prebois C, Katunuma N, Liaudet-Coopman E (2006) Processing of human cathepsin D is independent of its catalytic function and auto-activation: involvement of cathepsins L and B. J Biochem (Tokyo) 139: 363-371.
Laurent-Matha V, Farnoud MR, Lucas A, Rougeot C, Garcia M, Rochefort H (1998) Endocytosis of pro-cathepsin D into breast cancer cells is mostly independent of mannose-6-phosphate receptors. J Cell Sci 111: 2539-2549.
Laurent-Matha V, Lucas A, Huttler S, Sandhoff K, Garcia M, Rochefort H (2002) Procathepsin D interacts with prosaposin in cancer cells but its internalization is not mediated by LDL receptor-related protein. Exp Cell Res 277: 210-219.
Laurent-Matha V, Maruani-Herrmann S, Prebois C, Beaujouin M, Glondu M, Noel A, Alvarez-Gonzalez ML, Blacher S, Coopman P, Baghdiguian S., Gilles C, Loncarek J, Freiss G, Vignon F, Liaudet-Coopman E (2005) Catalytically inactive human cathepsin D triggers fibroblast invasive growth. J Cell Biol 168: 489-499.
Li Y, Lu W, Bu G (2003) Essential role of the low density lipoprotein receptor-related protein in vascular smooth muscle cell migration. FEBS Lett 555: 346-350.
Liaudet E, Derocq D, Rochefort H, Garcia M (1995) Transfected cathepsin D stimulates high density cancer cell growth by inactivating secreted growth inhibitors. Cell Growth Differ 6: 1045-1052.
Liaudet E, Garcia M, Rochefort H (1994) Cathepsin D maturation and its stimulatory effect on metastasis are prevented by addition of KDEL retention signal. Oncogene 9: 1145-1154.
Liaudet-Coopman E, Beaujouin M, Derocq D, Garcia M, Glondu-Lassis M, Laurent-Matha V, Prebois C, Rochefort H, Vignon F (2006) Cathepsin D: newly discovered functions of a long-standing aspartic protease in cancer and apoptosis. Cancer Lett 237: 167-179.
Lillis AP, Mikhailenko I, Strickland DK (2005) Beyond endocytosis: LRP function in cell migration, proliferation and vascular permeability. J Thromb Haemost 3: 1884-1893.
Liu CX, Ranganathan S, Robinson S, Strickland DK. gamma-Secretase-mediated release of the low density lipoprotein receptor-related protein 1B intracellular domain suppresses anchorage-independent growth of neuroglioma cells. J Biol Chem. 2007, 282:7504-11.
Loukinova E, Ranganathan S, Kuznetsov S, Gorlatova N, Migliorini MM, Loukinov D, Ulery PG, Mikhailenko I, Lawrence DA, Strickland DK (2002) Platelet-derived growth factor (PDGF)-induced tyrosine phosphorylation of the low density lipoprotein receptor-related protein (LRP). Evidence for integrated co-receptor function betwenn LRP and the PDGF. J Biol Chem 277: 15499-15506.
May P, Reddy YK, Herz J (2002) Proteolytic processing of low density lipoprotein receptor-related protein mediates regulated release of its intracellular domain. J Biol Chem 277: 18736-18743.
Metcalf P, Fusek M (1993) Two crystal structures for cathepsin D: the lysosomal targeting signal and active site. Embo J 12: 1293-1302.
Mohamed MM, Sloane BF (2006) Cysteine cathepsins: multifunctional enzymes in cancer. Nat Rev Cancer 6: 764-775.
Newton CS, Loukinova E, Mikhailenko I, Ranganathan S, Gao Y, Haudenschild C, Strickland DK (2005) Platelet-derived growth factor receptor-beta (PDGFR-beta) activation promotes its association with the low density lipoprotein receptor-related protein (LRP). Evidence for co-receptor function. J Biol Chem 280: 27872-27878.
Obermeyer K, Krueger S, Peters B, Falkenberg B, Roessner A, Rocken C. 2007. The expression of low density lipoprotein receptor-related protein in colorectal carcinoma. Oncol Rep. 17:361-7
Parton RG, Richards AA (2003) Lipid rafts and caveolae as portals for endocytosis: new insights and common mechanisms. Traffic 4: 724-738.
Quinn KA, Grimsley PG, Dai YP, Tapner M, Chesterman CN, Owensby DA (1997) Soluble low density lipoprotein receptor-related protein (LRP) circulates in human plasma. J Biol Chem 272: 23946-23951.
Quinn, K. A., Pye, V. J., Dai, Y. P., Chesterman, C. N., and Owensby, D. A. (1999). Characterization of the soluble form of the low density lipoprotein receptor-related protein (LRP). Exp Cell Res 251, 433-441.
Richo, G., and Conner, G. E. (1991). Proteolytic activation of human procathepsin D. Adv Exp Med Biol 306, 289-296.
Rochefort, H. (1992). Cathepsin D in breast cancer: a tissue marker associated with metastasis. Eur J Cancer 28A, 1780-1783.
Rochefort, H., and Liaudet-Coopman, E. (1999). Cathepsin D in cancer metastasis: a protease and a ligand. Apmis 107, 86-95.
Rodriguez, J., Vazquez, J., Corte, M. D., Lamelas, M., Bongera, M., Corte, M. G., Alvarez, A., Allende, M., Gonzalez, L., Sanchez, M., et al. (2005). Clinical significance of cathepsin D concentration in tumor cytosol of primary breast cancer. Int J Biol Markers 20, 103-111.
Rozanov, D. V., Hahn-Dantona, E., Strickland, D. K., and Strongin, A. Y. (2004). The low density lipoprotein receptor-related protein LRP is regulated by membrane type-1 matrix metalloproteinase (MT1-MMP) proteolysis in malignant cells. J Biol Chem 279, 4260-4268.
   Slentz-Kesler, K., Moore, J. T., Lombard, M., Zhang, J., Hollingsworth, R., and Weiner, M. P. (2000). Identification of the human Mnk2 gene (MKNK2) through protein interaction with estrogen receptor beta. Genomics 69, 63-71.
Strickland, D. K., and Ranganathan, S. (2003). Diverse role of LDL receptor-related protein in the clearance of proteases and in signaling. J Thromb Haemost 1, 1663-1670.
Vassar, R. et al. Beta-secretase cleavage of Alzheimer's amyloid precursor protein by the transmembrane aspartic protease BACE. Science 286,735-41 (1999).
Vetvicka, V., Vektvickova, J., and Fusek, M. (1994). Effect of human procathepsin D on proliferation of human cell lines. Cancer Lett 79, 131-135.
Vetvicka, V., Vetvickova, J., and Fusek, M. (1999). Anti-human procathepsin D activation peptide antibodies inhibit breast cancer development. Breast Cancer Res Treat 57, 261-269.
Vignon, F., Capony, F., Chambon, M., Freiss, G., Garcia, M., and Rochefort, H. (1986). Autocrine growth stimulation of the MCF 7 breast cancer cells by the estrogen-regulated 52 K protein. Endocrinology 118, 1537-1545.
Von Arnim, C. A., Kinoshita, A., Peltan, I. D., Tangredi, M. M., Herl, L., Lee, B. M., Spoelgen, R., Hshieh, T. T., Ranganathan, S., Battey, F. D., et al. (2005). The low density lipoprotein receptor-related protein (LRP) is a novel beta-secretase (BACE1) substrate. J Biol Chem 280, 17777-17785.
Von Figura, K., and Hasilik, A. (1986). Lysosomal enzymes and their receptors. Annu Rev Biochem 55, 167-193.
Westley, B. R., and May, F. E. (1999). Prognostic value of cathepsin D in breast cancer. Br J Cancer 79, 189-190.
Wu, L., and Gonias, S. L. (2005). The low-density lipoprotein receptor-related protein-1 associates transiently with lipid rafts. J Cell Biochem 96, 1021-1033.
Yang, M., Huang, H., Li, J., Li, D., and Wang, H. (2004). Tyrosine phosphorylation of the LDL receptor-related protein (LRP) and activation of the ERK pathway are required for connective tissue growth factor to potentiate myofibroblast differentiation. Faseb J 18, 1920-1921.
Zhang, H., Links, P. H., Ngsee, J. K., Tran, K., Cui, Z., Ko, K. W., and Yao, Z. (2004). Localization of low density lipoprotein receptor-related protein 1 to caveolae in 3T3-L1 adipocytes in response to insulin treatment. J Biol Chem 279, 2221-2230.

## Claims

1. An inhibitor of the interaction between pro-cath-D and the LRP1 β chain for the treatment and/or the prevention of a cancer secreting cath-D or of Alzheimer's disease, wherein said inhibitor is selected from the group consisting of a peptide having at least 80% sequence identity with SEQ ID NO:5, an anti-LRP1 β chain antibody, an antibody fragment which binds to the LRP1 β chain, and an aptamer which binds to the LRP1 β chain.

2. An inhibitor of the expression of LRP1 for the treatment and/or the prevention of a cancer secreting cath-D or of Alzheimer's disease.

3. The inhibitor of claim 2, wherein said inhibitor of LRP1 expression is selected from the group consisting of antisense RNA or DNA molecules, small inhibitory RNAs (siRNAs) and ribozymes.

4. The inhibitor according to claim 1, wherein said inhibitor is the peptide as set forth in SEQ ID NO: 5.

5. The inhibitor according to claim 1, wherein said inhibitor is an antibody or a fragment thereof specifically directed against the polypeptide F0 as set forth in SED ID NO: 3.

6. The inhibitor according to any one of claims 1 to 5, wherein said cancer secreting cath-D is selected from the group consisting of breast cancer, stomach cancer, liver cancer, glioblastoma, melanoma, squamous cell carcinoma, ovarian cancer and pancreatic cancer.

7. The inhibitor according to claim 6, wherein said cancer secreting cath-D is breast cancer.

8. An *in vitro* method for diagnosing a cancer secreting cath-D or Alzheimer's disease in a patient comprising detecting the presence of a fragment of LRP1 in a biological sample obtained from said patient.

9. The method according to claim 8, further comprising detecting the presence of pro-cath-D or a fragment thereof in a biological sample obtained from said patient.

10. The method according to claims 8 or 9 wherein said biological sample is a serum sample, a blood sample or a plasma sample.

11. Use of a fragment of LRP1 as a marker molecule for diagnosing a cancer secreting cath-D or Alzheimer's disease, wherein said fragment of LRP1 is selected from the group consisting of the LRP1 β chain ecto-domain as set forth in SEQ ID NO: 2, the LRP1 α chain and fragments thereof.

12. The method according to any one of claims 8 to 10 or the use according to claim 11, wherein said fragment of LRP1 is the LRP1 β chain ecto-domain as set forth in SEQ ID NO: 2 or a fragment thereof.

13. The method according to any one of claims 8 to 10, 12 or the use according to claim 11 or 12, wherein said cancer secreting cath-D is selected from the group consisting of breast cancer, stomach cancer, liver cancer, glioblastoma, melanoma, squamous cell carcinoma, ovarian cancer and pancreatic cancer.

14. The method according to claim 13 or the use according to claim 13, wherein said cancer secreting cath-D is breast cancer.

15. An inhibitor of the interaction between pro-cath-D and the β chain LRP1 for use in a method for treatment of the human or animal body by therapy, wherein said inhibitor is a peptide having at least 80% sequence identity with SEQ ID NO: 5.

16. An inhibitor of the interaction between pro-cath-D and the β chain LRP1 for use in a method for treatment of the human or animal body by therapy, wherein said inhibitor is an antibody or a fragment thereof directed against the polypeptide F0 as set forth in SEQ ID NO: 3.

17. An inhibitor of the interaction between pro-cath-D and the β chain LRP1 for use in a method for treatment of the human or animal body by therapy, wherein said inhibitor is an antibody or a fragment thereof directed against the peptide as set forth in SEQ ID NO: 5.

18. A method for the *in vitro* screening of compounds that inhibit the interaction between pro-cath-D and LRP1 β chain or a fragment thereof wherein said method comprises the following steps:
a) determining the ability of a candidate compound to inhibit the interaction between pro-cath-D and LRP1 β chain or a fragment thereof, and
b) selecting the candidate compound that inhibits the interaction between pro-cath-D and LRP1 β chain or a fragment thereof.

19. The method according to claim 18 wherein said fragment of the LPR1 β chain is selected from the group consisting of polypeptide F0 as set forth in SEQ ID NO: 3, polypeptide F4 as set forth in SEQ ID NO: 4 and peptide as set forth in SEQ ID NO: 5.


SEQUENCE LISTING

<110>  INSERM

<120>  Methods for treating and diagnosing a cancer secreting cath-D or
       Alzheimer's disease

<130>  BEP 070419

<160>  10

<170>  PatentIn version 3.3

<210>  1
<211>  601
<212>  PRT
<213>  Homo sapiens

<400>  1

Gln Ile Asp Arg Gly Val Thr His Leu Asn Ile Ser Gly Leu Lys Met
1               5                   10                  15

Pro Arg Gly Ile Ala Ile Asp Trp Val Ala Gly Asn Val Tyr Trp Thr
            20                  25                  30

Asp Ser Gly Arg Asp Val Ile Glu Val Ala Gln Met Lys Gly Glu Asn
        35                  40                  45

Arg Lys Thr Leu Ile Ser Gly Met Ile Asp Glu Pro His Ala Ile Val
    50                  55                  60

Val Asp Pro Leu Arg Gly Thr Met Tyr Trp Ser Asp Trp Gly Asn His
65                  70                  75                  80

Pro Lys Ile Glu Thr Ala Ala Met Asp Gly Thr Leu Arg Glu Thr Leu
                85                  90                  95

Val Gln Asp Asn Ile Gln Trp Pro Thr Gly Leu Ala Val Asp Tyr His
            100                 105                 110

Asn Glu Arg Leu Tyr Trp Ala Asp Ala Lys Leu Ser Val Ile Gly Ser
        115                 120                 125

Ile Arg Leu Asn Gly Thr Asp Pro Ile Val Ala Ala Asp Ser Lys Arg
    130                 135                 140

Gly Leu Ser His Pro Phe Ser Ile Asp Val Phe Glu Asp Tyr Ile Tyr
145                 150                 155                 160

Gly Val Thr Tyr Ile Asn Asn Arg Val Phe Lys Ile His Lys Phe Gly
                165                 170                 175

His Ser Pro Leu Val Asn Leu Thr Gly Gly Leu Ser His Ala Ser Asp
            180                 185                 190

Val Val Leu Tyr His Gln His Lys Gln Pro Glu Val Thr Asn Pro Cys

195                 200                 205

Asp Arg Lys Lys Cys Glu Trp Leu Cys Leu Leu Ser Pro Ser Gly Pro
    210                 215                 220

Val Cys Thr Cys Pro Asn Gly Lys Arg Leu Asp Asn Gly Thr Cys Val
225                 230                 235                 240

Pro Val Pro Ser Pro Thr Pro Pro Pro Asp Ala Pro Arg Pro Gly Thr
                245                 250                 255

Cys Asn Leu Gln Cys Phe Asn Gly Gly Ser Cys Phe Leu Asn Ala Arg
            260                 265                 270

Arg Gln Pro Lys Cys Arg Cys Gln Pro Arg Tyr Thr Gly Asp Lys Cys
        275                 280                 285

Glu Leu Asp Gln Cys Trp Glu His Cys Arg Asn Gly Gly Thr Cys Ala
    290                 295                 300

Ala Ser Pro Ser Gly Met Pro Thr Cys Arg Cys Pro Thr Gly Phe Thr
305                 310                 315                 320

Gly Pro Lys Cys Thr Gln Gln Val Cys Ala Gly Tyr Cys Ala Asn Asn
                325                 330                 335

Ser Thr Cys Thr Val Asn Gln Gly Asn Gln Pro Gln Cys Arg Cys Leu
            340                 345                 350

Pro Gly Phe Leu Gly Asp Arg Cys Gln Tyr Arg Gln Cys Ser Gly Tyr
        355                 360                 365

Cys Glu Asn Phe Gly Thr Cys Gln Met Ala Ala Asp Gly Ser Arg Gln
    370                 375                 380

Cys Arg Cys Thr Ala Tyr Phe Glu Gly Ser Arg Cys Glu Val Asn Lys
385                 390                 395                 400

Cys Ser Arg Cys Leu Glu Gly Ala Cys Val Val Asn Lys Gln Ser Gly
                405                 410                 415

Asp Val Thr Cys Asn Cys Thr Asp Gly Arg Val Ala Pro Ser Cys Leu
            420                 425                 430

Thr Cys Val Gly His Cys Ser Asn Gly Gly Ser Cys Thr Met Asn Ser
        435                 440                 445

Lys Met Met Pro Glu Cys Gln Cys Pro Pro His Met Thr Gly Pro Arg
    450                 455                 460

Cys Glu Glu His Val Phe Ser Gln Gln Gln Pro Gly His Ile Ala Ser
465                 470                 475                 480

Ile Leu Ile Pro Leu Leu Leu Leu Leu Leu Leu Val Leu Val Ala Gly
                485                 490                 495

Val Val Phe Trp Tyr Lys Arg Arg Val Gln Gly Ala Lys Gly Phe Gln
            500                 505                 510

His Gln Arg Met Thr Asn Gly Ala Met Asn Val Glu Ile Gly Asn Pro
        515                 520                 525

Thr Tyr Lys Met Tyr Glu Gly Gly Glu Pro Asp Asp Val Gly Gly Leu
    530                 535                 540

Leu Asp Ala Asp Phe Ala Leu Asp Pro Asp Lys Pro Thr Asn Phe Thr
545                 550                 555                 560

Asn Pro Val Tyr Ala Thr Leu Tyr Met Gly Gly His Gly Ser Arg His
                565                 570                 575

Ser Leu Ala Ser Thr Asp Glu Lys Arg Glu Leu Leu Gly Arg Gly Pro
            580                 585                 590

Glu Asp Glu Ile Gly Asp Pro Leu Ala
        595                 600

<210>  2
<211>  476
<212>  PRT
<213>  Homo sapiens

<400>  2

Gln Ile Asp Arg Gly Val Thr His Leu Asn Ile Ser Gly Leu Lys Met
1               5                   10                  15

Pro Arg Gly Ile Ala Ile Asp Trp Val Ala Gly Asn Val Tyr Trp Thr
            20                  25                  30

Asp Ser Gly Arg Asp Val Ile Glu Val Ala Gln Met Lys Gly Glu Asn
        35                  40                  45

Arg Lys Thr Leu Ile Ser Gly Met Ile Asp Glu Pro His Ala Ile Val
    50                  55                  60

Val Asp Pro Leu Arg Gly Thr Met Tyr Trp Ser Asp Trp Gly Asn His
65                  70                  75                  80

Pro Lys Ile Glu Thr Ala Ala Met Asp Gly Thr Leu Arg Glu Thr Leu
                85                  90                  95

Val Gln Asp Asn Ile Gln Trp Pro Thr Gly Leu Ala Val Asp Tyr His
            100                 105                 110

Asn Glu Arg Leu Tyr Trp Ala Asp Ala Lys Leu Ser Val Ile Gly Ser
        115                 120                 125

Ile Arg Leu Asn Gly Thr Asp Pro Ile Val Ala Ala Asp Ser Lys Arg
    130                 135                 140

Gly Leu Ser His Pro Phe Ser Ile Asp Val Phe Glu Asp Tyr Ile Tyr
145                 150                 155                 160

Gly Val Thr Tyr Ile Asn Asn Arg Val Phe Lys Ile His Lys Phe Gly
                165                 170                 175

His Ser Pro Leu Val Asn Leu Thr Gly Gly Leu Ser His Ala Ser Asp
            180                 185                 190

Val Val Leu Tyr His Gln His Lys Gln Pro Glu Val Thr Asn Pro Cys
        195                 200                 205

Asp Arg Lys Lys Cys Glu Trp Leu Cys Leu Leu Ser Pro Ser Gly Pro
    210                 215                 220

Val Cys Thr Cys Pro Asn Gly Lys Arg Leu Asp Asn Gly Thr Cys Val
225                 230                 235                 240

Pro Val Pro Ser Pro Thr Pro Pro Pro Asp Ala Pro Arg Pro Gly Thr
                245                 250                 255

Cys Asn Leu Gln Cys Phe Asn Gly Gly Ser Cys Phe Leu Asn Ala Arg
            260                 265                 270

Arg Gln Pro Lys Cys Arg Cys Gln Pro Arg Tyr Thr Gly Asp Lys Cys
        275                 280                 285

Glu Leu Asp Gln Cys Trp Glu His Cys Arg Asn Gly Gly Thr Cys Ala
    290                 295                 300

Ala Ser Pro Ser Gly Met Pro Thr Cys Arg Cys Pro Thr Gly Phe Thr
305                 310                 315                 320

Gly Pro Lys Cys Thr Gln Gln Val Cys Ala Gly Tyr Cys Ala Asn Asn
                325                 330                 335

Ser Thr Cys Thr Val Asn Gln Gly Asn Gln Pro Gln Cys Arg Cys Leu
            340                 345                 350

Pro Gly Phe Leu Gly Asp Arg Cys Gln Tyr Arg Gln Cys Ser Gly Tyr
        355                 360                 365

Cys Glu Asn Phe Gly Thr Cys Gln Met Ala Ala Asp Gly Ser Arg Gln
    370                 375                 380

Cys Arg Cys Thr Ala Tyr Phe Glu Gly Ser Arg Cys Glu Val Asn Lys

385                 390                 395                 400

Cys Ser Arg Cys Leu Glu Gly Ala Cys Val Val Asn Lys Gln Ser Gly
                405                 410                 415

Asp Val Thr Cys Asn Cys Thr Asp Gly Arg Val Ala Pro Ser Cys Leu
            420                 425                 430

Thr Cys Val Gly His Cys Ser Asn Gly Gly Ser Cys Thr Met Asn Ser
        435                 440                 445

Lys Met Met Pro Glu Cys Gln Cys Pro Pro His Met Thr Gly Pro Arg
    450                 455                 460

Cys Glu Glu His Val Phe Ser Gln Gln Gln Pro Gly
465                 470                 475

<210>  3
<211>  173
<212>  PRT
<213>  Artificial

<220>
<223>  fragment of the beta chain of LRP1

<400>  3

Pro Ser Gly Met Pro Thr Cys Arg Cys Pro Thr Gly Phe Thr Gly Pro
1               5                   10                  15

Lys Cys Thr Gln Gln Val Cys Ala Gly Tyr Cys Ala Asn Asn Ser Thr
            20                  25                  30

Cys Thr Val Asn Gln Gly Asn Gln Pro Gln Cys Arg Cys Leu Pro Gly
        35                  40                  45

Phe Leu Gly Asp Arg Cys Gln Tyr Arg Gln Cys Ser Gly Tyr Cys Glu
    50                  55                  60

Asn Phe Gly Thr Cys Gln Met Ala Ala Asp Gly Ser Arg Gln Cys Arg
65                  70                  75                  80

Cys Thr Ala Tyr Phe Glu Gly Ser Arg Cys Glu Val Asn Lys Cys Ser
                85                  90                  95

Arg Cys Leu Glu Gly Ala Cys Val Val Asn Lys Gln Ser Gly Asp Val
            100                 105                 110

Thr Cys Asn Cys Thr Asp Gly Arg Val Ala Pro Ser Cys Leu Thr Cys
        115                 120                 125

Val Gly His Cys Ser Asn Gly Gly Ser Cys Thr Met Asn Ser Lys Met
    130                 135                 140

Met Pro Glu Cys Gln Cys Pro Pro His Met Thr Gly Pro Arg Cys Glu
145                 150                 155                 160

Glu His Val Phe Ser Gln Gln Gln Pro Gly His Ile Ala
                165                 170

<210>  4
<211>  46
<212>  PRT
<213>  Artificial

<220>
<223>  fragment of the beta chain of LRP1

<400>  4

Cys Arg Cys Leu Pro Gly Phe Leu Gly Asp Arg Cys Gln Tyr Arg Gln
1               5                   10                  15

Cys Ser Gly Tyr Cys Glu Asn Phe Gly Thr Cys Gln Met Ala Ala Asp
            20                  25                  30

Gly Ser Arg Gln Cys Arg Cys Thr Ala Tyr Phe Glu Gly Ser
        35                  40                  45

<210>  5
<211>  49
<212>  PRT
<213>  Artificial

<220>
<223>  fragment of the beta chain of LRP1

<400>  5

Cys Arg Cys Leu Pro Gly Phe Leu Gly Asp Arg Cys Gln Tyr Arg Gln
1               5                   10                  15

Cys Ser Gly Tyr Cys Glu Asn Phe Gly Thr Cys Gln Met Ala Ala Asp
            20                  25                  30

Gly Ser Arg Gln Cys Arg Cys Thr Ala Tyr Phe Glu Gly Ser Arg Cys
        35                  40                  45

Glu

<210>  6
<211>  21
<212>  DNA
<213>  Artificial

<220>
<223>  target sequence

<400>  6
aagcagtttg cctgcagaga t                                                21

<210>  7

<211>  21
<212>  DNA
<213>  Artificial

<220>
<223>  target sequence

<400>  7
aagctatgag tttaagaagt t                                                 21


<210>  8
<211>  21
<212>  RNA
<213>  Artificial

<220>
<223>  target sequence

<400>  8
aagcaucuca guagacuauc a                                                 21


<210>  9
<211>  21
<212>  DNA
<213>  Artificial

<220>
<223>  target sequence

<400>  9
aagcagtttg cctgcagaga c                                                 21


<210>  10
<211>  21
<212>  DNA
<213>  Artificial

<220>
<223>  target sequence

<400>  10
aacgtacgcg gaatacttcg a                                                 21